(19) Europäisches Patentamt
European Patent Office
Office européen des brevets

(11) Numéro de publication: **0 463 944 B1**

# (12) FASCICULE DE BREVET EUROPEEN

(45) Date de publication de fascicule du brevet: **05.10.94**

(51) Int. Cl.⁵: **C07D 263/58**, A61K 31/42, C07D 413/06, C07D 263/52, C07D 263/60

(21) Numéro de dépôt: **91401678.7**

(22) Date de dépôt: **21.06.91**

(54) **Acyl benzoxazolinones, leur procédé de préparation et les compositions pharmaceutiques qui les contiennent.**

(30) Priorité: **22.06.90 FR 9007812**

(43) Date de publication de la demande:
**02.01.92 Bulletin 92/01**

(45) Mention de la délivrance du brevet:
**05.10.94 Bulletin 94/40**

(84) Etats contractants désignés:
**AT BE CH DE DK ES FR GB GR IT LI LU NL SE**

(56) Documents cités:
**EP-A- 0 049 203**
**FR-A- 2 244 506**

**EUROPEAN JOURNAL OF MEDICINAL CHEMISTRY-CHIMICA THERAPEUTICA, vol. 9, no. 5, septembre-octobre 1974, pages 491-496, Paris, FR; J.P. BONTE et al.: "Acyl-6 benzoxazolinones (1er mémoire)"**

**EUROPEAN JOURNAL OF MEDICINAL CHEMISTRY-CHIMICA THERAPEUTICA, vol. 9, no. 5, septembre-octobre 1974, pages 497-500, Paris, FR; J.P. BONTE et al.: "Acyl-6 benzoxazolinones (2me mémoire). Préparations de quelques produits detransformation"**

(73) Titulaire: **ADIR ET COMPAGNIE**
**1 rue Carle Hébert**
**F-92415 Courbevoie Cédex (FR)**

(72) Inventeur: **Aichaoui, Hocine**
**3, rue du Professeur Laguesse**
**F-59045 Lille Cedex (FR)**
Inventeur: **Lesieur, Daniel**
**20, rue de Verdun**
**F-59147 Gondecourt (FR)**
Inventeur: **Lespagnol, Charles**
**115, avenue Pasteur**
**F-59130 Lambersart (FR)**
Inventeur: **Devissaguet, Michelle**
**14, Boulevard d'Inkermann**
**F-92200 Neuilly Sur Seine (FR)**
Inventeur: **Guardiola, Béatrice**
**6, rue Edouard Nortier**
**F-92200 Neuilly Sur Seine (FR)**

**EP 0 463 944 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouvelles acyl benzoxazolinones, leur procédé de préparation et les compositions pharmaceutiques qui les contiennent.

De nombreux dérivés de la benzoxazolinone ont été décrits en thérapeutique.

Plus particulièrement les brevets Fr 73.23281, Fr 73.23280 ainsi que les publications Eur. J. Med. Chem. 1974, 9, (5), 491-496 et ibid 497-500 décrivent des acyl-6 benzoxazolinones analgésiques et antiinflammatoires.

La demanderesse a maintenant découvert des acyl benzoxazolinones douées d'une activité antithromboxane qui les différencie de l'art antérieur.

Plus spécifiquement l'invention concerne les dérivés de formule générale (I) :

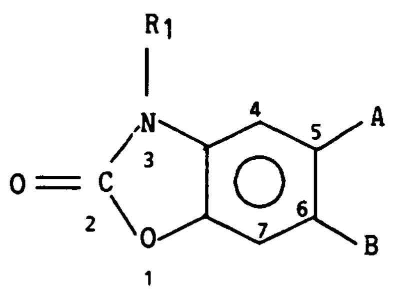

(I)

dans laquelle :

$R_1$ représente un atome d'hydrogène ou un groupement alkyle inférieur,

A représente un atome d'hydrogène et dans ce cas B représente un groupement CO-G, G étant :

- ou bien un groupement hétéroaryle choisi parmi furanne, indole, pyrrole, pyridine, quinoléine, isoquinoléine, benzofuranne éventuellement substitué par un ou plusieurs groupements alkyle inférieur ou alkoxy inférieur, ou atome d'halogène,
- ou bien un groupement alkyle inférieur linéaire ou ramifié substitué par un groupement carboxyle,
- ou bien un groupement alcényle inférieur linéaire ou ramifié substitué par un groupement carboxyle,
- ou bien un groupement phényle ou naphtyle substitué par un groupement carboxyle,
- ou bien A forme avec B un groupement $CO(CH_2)_nCH(CH_3)$ avec n nombre entier égal à 1, 2, 3 ou 4, le groupement CO étant fixé au cycle aromatique de la benzoxazolinone en position 5 ou 6, ainsi que le cas échéant, leurs isomères et lorsque le composé de formule (I) possède un groupement acide carboxylique, leurs sels d'addition à une base pharmaceutiquement acceptable et lorsque le composé de formule (I) comporte un groupement basique, leurs sels d'addition avec un acide pharmaceutiquement acceptable, étant entendu que alkyle inférieur, alkoxy inférieur et alcényle inférieur signifient des groupements linéaires ou ramifiés comprenant de 1 à 6 atomes de carbone.

Parmi les acides pharmaceutiquement acceptables que l'on peut, le cas échéant, ajouter aux composés de formule (I) renfermant un groupement basique pour obtenir un sel, on peut citer à titre non limitatif, les acides chlohydrique, sulfurique, tartrique, maléique, fumarique, oxalique, méthane-sulfonique, camphorique, etc...

Parmi les bases pharmaceutiquement acceptables que l'on peut, le cas échéant, ajouter aux composés de formule (I) renfermant un groupement acide carboxylique pour obtenir un sel, on peut citer à titre non limitatif les hydroxydes de sodium, potassium, calcium ou d'aluminium, des carbonates de métaux alcalins ou alcalinoterreux, ou des bases organiques comme la triéthylamine, la benzylamine, la diéthanolamine, la tert.butylamine, la dicyclohexylamine, l'arginine, etc...

La présente invention a également pour objet le procédé de préparation des composés de formule (I) caractérisé en ce que l'on utilise comme matière première un dérivé de formule (II) :

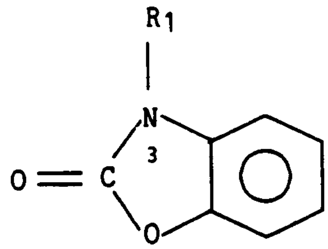

(II)

dans laquelle $R_1$ a la même définition que dans la formule (I),
que l'on traite en présence de diméthylformamide et de chlorure d'aluminium :

a) soit :

- par un chlorure d'acide de formule (III) :

**ClCOG (III)**

dans laquelle :
G a la même définition que dans la formule (I) à l'exception du cas où G représente un groupement alcényle inférieur substitué par un carboxyle,

- ou par un anhydride d'acide de formule (IV) :

**$O(OCG)_2$ (IV)**

dans laquelle G a la même définition que dans la formule (I),
pour obtenir un dérivé de formule (I/a) :

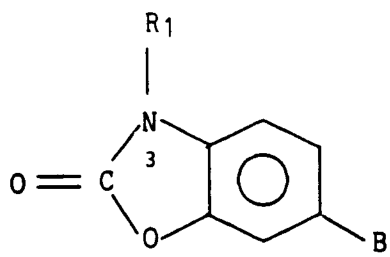

(I/a)

cas particulier des dérivés de formule (I) dans lequel A représente un atome d'hydrogène et B a la même signification que dans la formule (I) lorsque A représente un atome d'hydrogène,
dérivé de formule (I/a) que l'on purifie si nécessaire par une technique classique choisie parmi cristallisation ou chromatographie, dont on sépare le cas échéant les isomères et que l'on salifie si on le désire, lorsque B renferme un groupement acide carboxylique par une base pharmaceutiquement acceptable et lorsque B renferme un groupement basique par un acide pharmaceutiquement acceptable,
b) soit par un chlorure d'acide de formule (V) :

**$ClOC-(CH_2)_{n-1}CH=CH-CH_3$ (V)**

dans laquelle n a la même définition que dans la formule (I),
soit par un dérivé de formule :

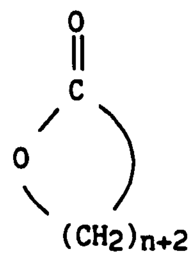

dans laquelle n a la même définition que dans la formule (I),
pour obtenir un mélange de dérivés de formule (I/b) :

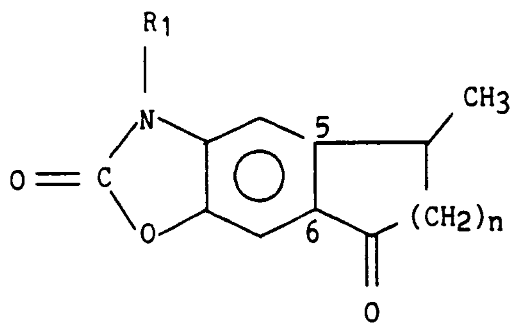

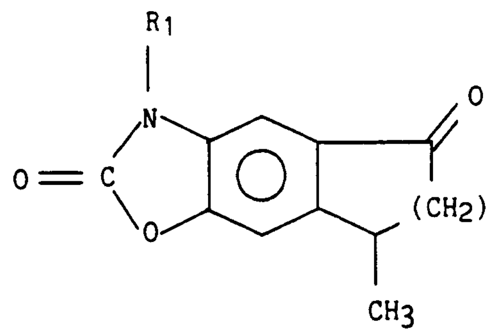

(I/b)

dans laquelle $R_1$ et n ont la même définition que dans la formule (I),
cas particulier de dérivés de formule (I) dans lesquels A forme avec B un groupement $CO(CH_2)_nCHCH_3$, dans laquelle n à la même définition que dans la formule (I), le groupement CO étant fixé soit en position 5, soit en position 6 de la benzoxazolinone, dérivés de formule (I/b) que l'on sépare et purifie par une ou plusieurs techniques choisies parmi cristallisation ou chromatographie.

Un cas particulier de dérivé de la présente invention concerne les dérivés de formule (I/c) :

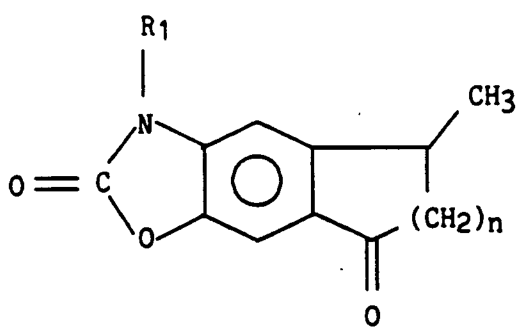

(I/c)

dans lesquels $R_1$ et n ont la même définition que dans la formule (I) que l'on peut obtenir par action sur un dérivé de formule (IV) :

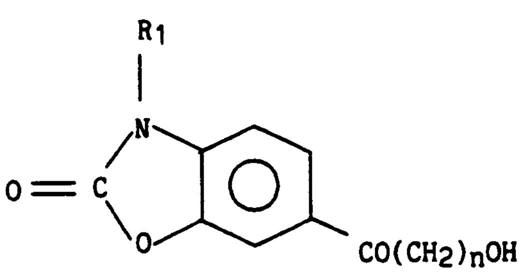

(IV)

dans lequel $R_1$ et n ont la même définition que dans la formule (I),
du chlorure d'aluminium en présence de diméthylformamide,
pour obtenir un dérivé de formule (I/c) que l'on purifie si on le désire, par une ou plusieurs techniques choisies parmi cristallisation et/ou chromatographie.

Les composés de formule (I) possèdent des propriétés pharmacologiques intéressantes.

L'étude pharmacologique des dérivés de l'invention a en effet montré qu'ils étaient peu toxiques, doués d'activité antithromboxane.

Ce spectre d'activité rend donc les composés de la présente invention intéressants dans un certain nombre d'indications telles que dans la prévention des accidents ischémiques artériels périphériques et cérébro vasculaires.

La présente invention a également pour objet les compositions pharmaceutiques contenant les produits de formule (I), seuls ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, les comprimés simples ou dragéifiés, les comprimés sublingaux, les sachets, les paquets, les gélules, les glossettes, les tablettes, les suppositoires, les crèmes, pommades, gels dermiques etc...

La posologie utile varie selon l'âge et le poids du patient, la voie d'administration, la nature de l'indication thérapeutique ou des traitements éventuellement associés et s'échelonne entre 0,5 centigramme et 4 grammes par 24 heures.

Les exemples suivants illustrent l'invention et ne la limitent en aucune façon.

Les spectres infrarouge sont réalisés en pastille de bromure de potassium.

**EXEMPLE 1 : 3-METHYL 6-NICOTINOYL BENZOXAZOLINONE**

Dans une fiole de 250 ml contenant 61,3 g (0,46 mole) de chlorure d'aluminium, introduire goutte à goutte et sous agitation 9,9 ml (0,13 mole) de diméthylformamide.
Munir la fiole d'un réfrigérant à reflux et porter dans un bain d'huile à une température voisine de 45 °C.
Introduire 6 g (0,04 mole) de 3-méthyl benzoxazolinone et 10,6 g (0,06 mole) de chlorhydrate de chlorure d'acide nicotinique.
Chauffer à la température t (100-110 °C) pendant un temps $\theta$ (80 heures).
Verser le mélange réactionnel dans une quantité suffisante de glace, agiter une heure, essorer le précipité formé, laver à l'eau et sécher. Recristalliser dans l'éthanol.
Rendement : 68%
Point de fusion : 163-164 °C

| Analyse élémentaire : | | | |
|---|---|---|---|
| Calculée<br>Trouvée | C : 66,13<br>C : 66,24 | H : 3,96<br>H : 3,96 | N : 11,02<br>N : 10,87 |

Caractéristiques spectrales :
Infrarouge :
1770 $cm^{-1}$ : $\nu$ C = O (OCON)
1635 $cm^{-1}$ : $\nu$ C = O (cétonique)
1600-1580 $cm^{-1}$ : $\nu$ C = C (aromatique)
Résonance magnétique nucléaire (Solvant DMSO $D_6$)
$\delta$ : 3,08 ppm, singulet 3H N-$CH_3$

**EXEMPLE 2 : 6-NICOTINOYL BENZOXAZOLINONE (CHLORHYDRATE)**

Procéder comme dans l'exemple 1 en remplaçant la 3-méthyl benzoxazolinone par la benzoxazolinone.
$\theta$ : 30 heures
Rendement : 60%
Point de fusion : 259-260 °C

| Analyse élémentaire : | | | |
|---|---|---|---|
| Calculée<br>Trouvée | C : 56,43<br>C : 56,30 | H : 3,28<br>H : 3,35 | N : 10,12<br>N : 10,00 |

Caractéristiques spectrales :
Infrarouge :
1775 $cm^{-1}$ : ν CO (OCON)
1645 $cm^{-1}$ : ν CO (cétonique)
1610-1580 $cm^{-1}$ : ν C=C (aromatique)
Résonance magnétique nucléaire (Solvant DMSO $D_6$)
δ : 7,26 ppm, doublet (1H), $H_4$, J = 8,8Hz
δ : 6,68 ppm, doublet (1H), $H_7$, J = 2,2Hz
δ : 6,70 ppm, doublet dédoublé (1H), $H_5$, J = 8,8Hz ; J = 2,2Hz

**EXEMPLE 3 : 6-FUROYL 3-METHYL BENZOXAZOLINONE**

En procédant comme dans l'exemple 1, mais en remplaçant le chlohydrate de chlorure d'acide nicotinique par le chlorure de 2-furoyle, on obtient le produit du titre.
t : 95-100 °C
θ : 4H30
Rendement : 72%
Point de fusion : 140-141 °C

| Analyse élémentaire : | | | |
|---|---|---|---|
| Calculée<br>Trouvée | C : 64,19<br>C : 64,26 | H : 3,67<br>H : 3,63 | N : 5,67<br>N : 5,86 |

Caractéristiques spectrales :
Infrarouge :
1760 $cm^{-1}$ : ν CO (OCON)
1625 $cm^{-1}$ : ν CO (cétonique)
1600 $cm^{-1}$ : ν C=C (aromatique)
Résonance magnétique nucléaire (Solvant DMSO $D_6$)
δ : 3,41 ppm, singulet 3H, N-$CH_3$
δ : 7,40 ppm, doublet, 1H, ($H_4$), J = 7,7Hz
δ : 7,84 ppm, singulet, 1H, ($H_7$)
δ : 7,94 ppm, doublet dédoublé, 1H, ($H_5$), J = 7,7 Hz ; J = 1,6Hz

**EXEMPLE 4 : 6-FUROYL BENZOXAZOLINONE**

En procédant comme dans l'exemple 2, mais en remplaçant le chlorhydrate du chlorure de l'acide nicotinique par le chlorure de 2-furoyle, on obtient le produit du titre.
t : 95-100 °C
θ : 5H
Rendement : 68%
Point de fusion : 233-234 °C

| Analyse élémentaire : | | | |
|---|---|---|---|
| Calculée<br>Trouvée | C : 62,88<br>C : 62,55 | H : 3,08<br>H : 3,11 | N : 6,11<br>N : 5,97 |

Caractéristiques spectrales :
Infrarouge :
3100 $cm^{-1}$ : ν NH

1775 cm$^{-1}$ : $\nu$ CO (OCON)
1620 cm$^{-1}$ : $\nu$ CO (cétonique)
1583 cm$^{-1}$ : $\nu$ C=C (aromatique)
Résonance magnétique nucléaire $^1$H(Solvant DMSO $D_6$)
$\delta$ : 7,24 ppm, doublet, 1H, (H4), J = 7,6Hz
$\delta$ : 7,80 ppm, singulet, 1H, $H_7$
$\delta$ : 7,85 ppm, doublet dédoublé, 1H, $H_5$, J = 7,6Hz

**EXEMPLE 5 : ACIDE 4-OXO 4-(3-METHYL BENZOXAZOLINON-6-YL) BUTYRIOUE**

Dans une fiole de 250 ml contenant 53,3 g (0,4 mole) de chlorure d'aluminium, introduire goutte à goutte et sous agitation 8,6 ml (0,115 mole) de diméthylformamide. Munir la fiole d'un réfrigérant à reflux et porter dans un bain d'huile à une température voisine de 45°C.
Introduire 6 g (0,04 mole) de 3-méthyl benzoxazolinone et 6 g d'anhydride succinique (0,06 mole). Chauffer à 90-95°C (température t) pendant un temps $\theta$(5H30 minutes).
Verser le mélange réactionnel dans une quantité suffisante de glace, agiter une heure et essorer le précipité formé. Reprendre le produit obtenu par une solution aqueuse de bicarbonate de sodium à 10%. Extraire la solution alcaline à plusieurs reprises à l'éther puis acidifier la phase aqueuse à l'acide chlorhydrique dilué. Essorer le précipité obtenu, laver à l'eau, sécher et recristalliser dans l'éthanol.
Rendement : 62%
Point de fusion : 179-180°C

| Analyse élémentaire : | | | |
|---|---|---|---|
| Calculée | C : 57,83 | H : 4,44 | N : 5,62 |
| Trouvée | C : 57,62 | H : 4,53 | N : 5,83 |

Caractéristiques spectrales :
Infrarouge :
3300 cm$^{-1}$ : $\nu$ OH (acide)
1750 cm$^{-1}$ : $\nu$ CO (N-CO-O)
1730 cm$^{-1}$ : $\nu$ CO (acide)
1660 cm$^{-1}$ : $\nu$ CO (cétonique)
1600 cm$^{-1}$ : $\nu$ C=C (aromatique)
Résonance magnétique nucléaire (Solvant : acétone D6)
$\delta$ : 3,36 ppm, triplet, 2H, CO-$CH_2$
$\delta$ : 3,50 ppm, singulet, 3H, N-$CH_3$

**EXEMPLE 6 : ACIDE 4-OXO 4-(BENZOXAZOLINON-6 YL) BUTYRIOUE**

En procédant comme dans l'exemple 5, mais en remplaçant la 3-méthyl benzoxazolinone par la benzoxazolinone, on obtient le produit du titre.
t : 90-95°C
$\theta$ : 5H30
Solvant de recristallisation : Ethanol/eau (1:4)
Rendement : 54%
Point de fusion : 218-219°C
Caractéristiques spectrales :
Infrarouge :
3290 cm$^{-1}$ : $\nu$ OH (acide)
3060 cm$^{-1}$ : $\nu$ NH
1740 cm$^{-1}$ : $\nu$ CO (OCON)
1670 cm$^{-1}$ : $\nu$ CO (cétonique)
1705 cm$^{-1}$ : $\nu$ CO (acide)
Résonance magnétique nucléaire (Solvant : acétone D6)
$\delta$ : 2,74 ppm, triplet, 2H, $CH_2$-COOH
$\delta$ : 3,33 ppm, triplet, 2H, CO-$CH_2$

**EXEMPLE 7 : ACIDE 2-METHYL 4-OXO 4-(3-METHYL BENZOXAZOLINON-6-YL) BUTYRIOUE**

En procédant comme dans l'exemple 5, mais en remplaçant l'anhydride succinique par l'anhydride méthyl succinique, on obtient le produit du titre.
Température t : 80-85 ° C
θ : 4H30
Solvant de recristallisation : eau : éthanol (4:2)
Rendement : 43%
Point de fusion : 186-187 ° C

| Analyse élémentaire : | | | |
|---|---|---|---|
| Calculée<br>Trouvée | C : 59,31<br>C : 59,17 | H : 4,98<br>H : 4,90 | N : 5,32<br>N : 5,39 |

Caractéristiques spectrales :
Infrarouge :
3060-2880 $cm^{-1}$ : ν OH (acide)
1770 $cm^{-1}$ : ν CO (OCON)
1700 $cm^{-1}$ : ν CO (acide)
1670 $cm^{-1}$ : ν CO (cétonique)
Résonance magnétique nucléaire (Solvant : DMSO $D_6$)
δ : 1,18 ppm, doublet, 3H, $CH_3$CH-COOH
δ : 2,90 à 3,64 ppm, massif, 6H, N-$CH_3$ et $CH_2$-CH-COOH

**EXEMPLE 8 : ACIDE 2-METHYL 4-OXO 4-(BENZOXAZOLINON-6-YL) BUTYRIOUE**

En procédant comme dans l'exemple 7, mais en remplaçant la 3-méthyl benzoxazolinone par la benzoxazolinone, on obtient le produit du titre.
Température t : 80-85 ° C
θ : 4H3O
Solvant de recristallisation : eau/éthanol (5/0,5)
Rendement : 32%
Point de fusion : 206-208 ° C

| Analyse élémentaire : | | | |
|---|---|---|---|
| Calculée<br>Trouvée | C : 57,83<br>C : 57,61 | H : 4,45<br>H : 4,35 | N : 5,62<br>N : 5,52 |

Caractéristiques spectrales :
Infrarouge :
3210-2900 $cm^{-1}$ : ν NH et OH (acide)
1760 $cm^{-1}$ : ν CO (OCON)
1690 $cm^{-1}$ : ν CO (acide)
1675 $cm^{-1}$ : ν CO (cétonique)
Résonance magnétique nucléaire (Solvant : DMSO $D_6$)
δ : 1,19 ppm, doublet, 3H,-$CH_3$-CH-COOH
δ : 2,73 à 3,62 ppm, massif, 3H, $CH_2$-CH-COOH

**EXEMPLE 9 : ACIDE 4-OXO 4-(3-METHYL BENZOXAZOLINON-6-YL)BUTENE-2-OÏOUE**

En procédant comme dans l'exemple 5, mais en remplaçant l'anhydride succinique par l'anhydride maléique, on obtient le produit du titre.
Température t : 80 ° C
θ : 4H30
Solvant de recristallisation : EtOH

Rendement : 58%
Point de fusion : 220-221 °C

| Analyse élémentaire : | | | |
|---|---|---|---|
| Calculée | C : 58,30 | H : 3,67 | N : 5,67 |
| Trouvée | C : 58,06 | H : 3,88 | N : 5,65 |

Caractéristiques spectrales :
Infrarouge :
3280 $cm^{-1}$ : ν OH (acide)
1760 $cm^{-1}$ : ν CO (NCOO)
1720 $cm^{-1}$ : ν CO (acide)
1655 $cm^{-1}$ : ν CO (cétonique)
Résonance magnétique nucléaire (Solvant : DMSO $D_6$)
δ : 3,41 ppm, singulet, 3H, N-$CH_3$

**EXEMPLE 10 : ACIDE 4-OXO 4-(BENZOXAZOLINON-6-YL) BUTENE-2-OÏOUE**

En procédant comme dans l'exemple 9, mais en remplaçant la 3-méthyl benzoxazolinone par la benzoxazolinone, on obtient le produit du titre.
Température t : 80 °C
θ : 4H30
Solvant de recristallisation : eau
Rendement : 49%
Point de fusion : 235-236 °C

| Analyse élémentaire : | | | |
|---|---|---|---|
| Calculée | C : 56,65 | H : 3,03 | N : 6,00 |
| Trouvée | C : 56,69 | H : 3,25 | N : 6,03 |

Caractéristiques spectrales :
Infrarouge :
3220 $cm^{-1}$ : ν OH (acide)
3000 $cm^{-1}$ : ν NH
1755 $cm^{-1}$ : ν CO (OCON)
1700 $cm^{-1}$ : ν CO (acide)
1650 $cm^{-1}$ : ν CO (cétonique)
Résonance magnétique nucléaire (Solvant : DMSO $D_6$)
δ : 7,80 ppm, doublet, 1H, $H_4$, $J_{4-5}$ = 7,5Hz
δ : 7,85 ppm, singulet, 1H,$H_7$
δ : 7,90 ppm, doublet, 1H, $H_5$, $J_{5-4}$ = 7,5Hz

**EXEMPLE 11 : ACIDE 2-METHYLENE 4-OXO 4-(3-METHYL BENZOXAZOLINON-6-YL)BUTYRIOUE**

En procédant comme dans l'exemple 5, mais en remplaçant l'anhydride succinique par l'anhydride itaconique, on obtient le produit du titre.
Température t : 75-80 °C
θ : 4H30
Rendement : 46%
Solvant de recristallisation : éthanol
Point de fusion : 180 °C

| Analyse élémentaire : | | | |
|---|---|---|---|
| Calculée<br>Trouvée | C : 59,77<br>C : 59,96 | H : 4,24<br>H : 4,19 | N : 5,36<br>N : 5,84 |

Caractéristiques spectrales :
Infrarouge :
3100 à 2800 $cm^{-1}$ : $\nu$ OH (acide)
1745 $cm^{-1}$ : $\nu$ NH
1710 $cm^{-1}$ : $\nu$ CO (acide)
1670 $cm^{-1}$ : $\nu$ CO (cétonique)
Résonance magnétique nucléaire (Solvant : DMSO $D_6$)
$\delta$ : 3,34 ppm, singulet, 3H, N-$CH_3$
$\delta$ : 4,02 ppm, singulet, 2H, CO-$CH_2$
$\delta$ : 7,90 ppm, doublet, 1H, $H_5$, $J_{5-4}$ = 7,5Hz

**EXEMPLE 12 : ACIDE 2-METHYLENE 4-OXO 4-(BENZOXAZOLINON-6-YL) BUTYRIOUE**

En procédant comme dans l'exemple 11, mais en remplaçant la 3-méthyl benzoxazolinone par la benzoxazolinone, on obtient le produit du titre.
Température t : 75-80 ° C
$\theta$ : 5H
Solvant de recristallisation : eau : éthanol (1/1)
Rendement : 34%
Point de fusion : 222-223 ° C

| Analyse élémentaire : | | | |
|---|---|---|---|
| Calculée<br>Trouvée | C : 58,30<br>C : 58,79 | H : 3,67<br>H : 3,38 | N : 5,67<br>N : 5,20 |

Caractéristiques spectrales :
Infrarouge :
3300 $cm^{-1}$ : $\nu$ OH (acide)
3080 $cm^{-1}$ : $\nu$ NH
1745 $cm^{-1}$ : $\nu$ CO (OCON)
1695 $cm^{-1}$ : $\nu$ CO (acide)
1615 $cm^{-1}$ : $\nu$ CO (cétonique)
1605 $cm^{-1}$ : $\nu$ C = C (aromatiuqe)
Résonance magnétique nucléaire (Solvant : DMSO $D_6$)
$\delta$ : 7,18 ppm, doublet, 1H, $H_4$, $J_{4-5}$ = 8,4Hz
$\delta$ : 7,81 ppm, singulet, 1H, $H_7$
$\delta$ : 7,87 ppm, doublet, 1H, $H_5$, $J_{5-4}$ = 8,4Hz

**EXEMPLE 13 : ACIDE 2-[(3-METHYL BENZOXAZOLINON-6-YL)CARBONYL]BENZOÏOUE**

En procédant comme dans l'exemple 5, mais en remplaçant l'anhydride succinique par l'anhydride phtalique, on obtient le produit du titre.
Température t : 95-100 ° C
$\theta$ : 8H
Solvant de recristallisation : éthanol
Rendement : 58%
Point de fusion : 210-211 ° C

| Analyse élémentaire : | | | |
|---|---|---|---|
| Calculée<br>Trouvée | C : 64,63<br>C : 64,14 | H : 3,73<br>H : 3,92 | N : 4,71<br>N : 4,74 |

Caractéristiques spectrales :
Infrarouge :
3460 $cm^{-1}$ : $\nu$ OH
1775 $cm^{-1}$ : $\nu$ CO (OCON)
1680 $cm^{-1}$ : $\nu$ CO (acide)
1640 $cm^{-1}$ : $\nu$ CO (cétonique)
Résonance magnétique nucléaire (Solvant : DMSO $D_6$)
$\delta$ : 3,36 ppm, 3H, N-$CH_3$
$\delta$ : 7,26 ppm, doublet, 1H, ($H_4$), $J_{4-5}$ : 8,7Hz
$\delta$ : 7,68 ppm, doublet dédoublé, 1H, ($H_5$), $J_{5-4}$ = 8,7Hz

**EXEMPLE 14 : ACIDE 2-(BENZOXAZOLINON-6-YL CARBONYL) BENZOÏQUE**

En procédant comme dans l'exemple 13, mais en remplaçant la méthyl-3 benzoxazolinone par la benzoxazolinone, on obtient le produit du titre.
Température t : 95-100°C
$\theta$: 8H
Solvant de recristallisation : eau/éthanol (3/1)
Rendement : 52%
Point de fusion : 243-244°C

| Analyse élémentaire : | | | |
|---|---|---|---|
| Calculée<br>Trouvée | C : 63,60<br>C : 63,29 | H : 3,20<br>H : 3,18 | N : 4,94<br>N : 4,92 |

Caractéristiques spectrales :
Infrarouge :
3470 $cm^{-1}$ : $\nu$ OH (acide)
3360 $cm^{-1}$ : $\nu$ NH
1770 $cm^{-1}$ : $\nu$ CO (OCON)
1690 $cm^{-1}$ : $\nu$ CO (acide)
1650 $cm^{-1}$ : $\nu$ CO (cétonique)
Résonance magnétique nucléaire (Solvant : DMSO $D_6$)
$\delta$ : 7,15 ppm, doublet, 1H ($H_4$), $J_{4-5}$ = 8,5Hz
$\delta$ : 7,70 ppm, doublet dédoublé, 1H, ($H_5$), $J_{5-4}$ = 8,5Hz

**EXEMPLE 15 : 2,3-DIHYDRO 2,5-DIOXO 3,7-DIMETHYL CYCLOPENTA [f] BENZOXAZOLE**

En procédant comme dans l'exemple 5, mais en remplaçant l'anhydride succinique par le chlorure d'acide crotonique ou encore la $\gamma$ butyrolactone, on obtient le produit du titre.
Purifier par chromatographie sur colonne de gel de silice par le mélange cyclohexane / acétate d'éthyle 1/5.
Température t : 80-85°C
$\theta$: 2H30 à 3H
Solvant de recristallisation : éthanol
Rendement : 52%
Point de fusion : 166-167°C

| Analyse élémentaire : | | | |
|---|---|---|---|
| Calculée<br>Trouvée | C : 66,35<br>C : 66,18 | H : 5,10<br>H : 5,17 | N : 6,45<br>N : 6,39 |

Caractéristiques spectrales :
Infrarouge :
1770 $cm^{-1}$ : ν CO (OCON)
1680 $cm^{-1}$ : ν CO (cétonique)
Résonance magnétique nucléaire (Solvant : DMSO $D_6$) :
δ : 1,34 ppm, doublet, 3H, HC-$CH_3$, J = 6,6Hz
δ : 3,36 ppm, singulet, 3H, N-$CH_3$

**EXEMPLE 16 : 2,3-DIHYDRO 2,7 DIOXO 3,5-DIMETHYL CYCLOPENTA [f] BENZOXAZOLE**

Dans une fiole de 250 ml contenant 53,3 g (0,4 mole) de chlorure d'aluminium, introduire goutte à goutte et sous agitation 8,6 ml (0,115 mole) de diméthylformamide. Munir la fiole d'un réfrigérant à reflux et porter dans un bain d'huile à une température voisine de 45°C. introduire 6 g (0,04 mole) de 6-(4-hydroxy butyryl)-3-méthyl benzoxazolinone et chauffer à 90°C pendant 2 heures.
Hydrolyser le milieu réactionnel et procéder comme décrit dans l'exemple 5.
Rendement : 52%
Point de fusion : 189°C
Solvant de recristallisation : éthanol

| Analyse élémentaire : | | | |
|---|---|---|---|
| Calculée<br>Trouvée | C : 66,35<br>C : 66,19 | H : 5,10<br>H : 5,21 | N : 6,45<br>N : 6,43 |

Caractéristiques spectrales :
Infrarouge :
1770 $cm^{-1}$ : ν CO (OCON)
1695 $cm^{-1}$ : ν CO (cétonique)
Résonance magnétique nucléaire (Solvant : DMSO $D_6$)
δ : 1,38 ppm, doublet, 3H, CH-$CH_3$, J = 7Hz
δ : 3,39 ppm, singulet, 3H, N-$CH_3$
NB. : Ce composé peut être obtenu en utilisant le même mode opératoire que celui décrit dans l'exemple 15. Les deux composés sont séparés par chromatographie, l'exemple 16 étant élué en second.

**EXEMPLE 17 : 2,3-DIHYDRO 2,5-DIOXO 7-METHYL CYCLOPENTA [f] BENZOXAZOLE**

En procédant comme dans l'exemple 15, mais en remplaçant la 3-méthyl benzoxazolinone par la benzoxazolinone, on obtient le produit du titre.
Solvant de recristallisation : (EtOH:eau)(2:4)
Point de fusion : 252-253°C

| Analyse élémentaire : | | | |
|---|---|---|---|
| Calculée<br>Trouvée | C : 65,02<br>C : 64,77 | H : 4,47<br>H : 4,37 | N : 6,89<br>N : 6,84 |

Caractéristiques spectrales :
Infrarouge :
1770 $cm^{-1}$ : ν CO (O-CO-N)
1680 $cm^{-1}$ : ν CO (cétonique)
Résonance magnétique nuclaire (Solvant : DMSO $D_6$)
δ : 1,36 ppm, doublet, 3H, CH-$CH_3$

**EXEMPLE 18 : 2,3-DIHYDRO 2,7-DIOXO 5-METHYL CYCLOPENTA [f] BENZOXAZOLE**

En procédant comme dans l'exemple 16 et en remplaçant la 6-(4-hydroxy butyryl) 3-méthyl benzoxazolinone par la 6-(4-hydroxy butyryl)benzoxazolinone, on obtient le produit du titre.
Point de fusion : 213-214 °C
Solvant de recristallisation : éthanol / eau 1/2 (v/v) (2:4)

| Analyse élémentaire : | | | |
|---|---|---|---|
| Calculée | C : 65,02 | H : 4,47 | N : 6,89 |
| Trouvée | C : 64,72 | H : 4,70 | N : 6,98 |

Caractéristiques spectrales :
Infrarouge :
1780 $cm^{-1}$ : ν CO (OCON)
1775 $cm^{-1}$ : ν CO (cétonique)
Résonance magnétique nucléaire (Solvant : DMSO $D_6$) :
δ : 1,32 ppm, doublet, 3H, C-$CH_3$, J = 6,6Hz
NB : Ce composé peut être obtenu en utilisant le même mode opératoire que celui décrit pour l'exemple 17. Les deux composés sont séparés par chromatographie. L'exemple 18 étant élué en second.

**EXEMPLE 19 : 2,3-DIHYDRO 8-METHYL 2,5-DIOXO CYCLOHEXA [f] BENZOXAZOLE**

En procédant comme dans l'exemple 17, mais en remplaçant le chlorure d'acide crotonique ou la γ butyrolactone par la δ valérolactone, on obtient le produit du titre.
Purification par chromatographie sur colonne de gel de silice avec le mélange cyclohexane / acétate d'éthyle (1:5).
Température t : 90-95 °C
Temps de chauffage : 4H - 4H30
Point de fusion : 244-245 °C
Rendement : 80%
Solvant de recristallisation : éthanol

| Analyse élémentaire : | | | |
|---|---|---|---|
| Calculée | C : 66,35 | H : 5,10 | N : 6,44 |
| Trouvée | C : 66,13 | H : 4,92 | N : 6,86 |

Caractéristiques spectrales :
Infrarouge :
3300 $cm^{-1}$ : ν NH
1775 $cm^{-1}$ : ν CO (OCON)
1660 $cm^{-1}$ : ν CO (cétonique)
Résonance magnétique nucléaire (Solvant : DMSO $D_6$)
δ : 1,35 ppm, doublet, 3H, C-$CH_3$, J = 6,5Hz

**EXEMPLE 20 : 2,3-DIHYDRO 5-METHYL 2,8-DIOXO CYCLOHEXA [f] BENZOXAZOLE**

En utilisant le mode opératoire décrit dans l'exemple 19, on obtient le produit du titre lors de la purification par chromatographie.
Point de fusion : 247-248 °C
Rendement : 20%

| Analyse élémentaire : | | | |
|---|---|---|---|
| Calculée<br>Trouvée | C : 66,35<br>C : 66,57 | H : 5,10<br>H : 5,54 | N : 6,44<br>N : 6,09 |

Caractéristiques spectrales :
Infrarouge :
3300 $cm^{-1}$ : ν NH
1780 $cm^{-1}$ : ν CO (OCON)
1650 $cm^{-1}$ : ν CO (cétonique)
Résonance magnétique nucléaire (Solvant : DMSO $D_6$)
δ : 1,36 ppm, doublet, 3H, C-$CH_3$, J = 6,7Hz

**EXEMPLE 21 : 2,3-DIHYDRO 3,8- DIMETHYL 2,5-DIOXO CYCLOHEXA [f] BENZOXAZOLE**

Procéder comme dans l'exemple 19 en remplaçant la benzoxazolinone par la 3-méthyl benzoxazolinone.
Solvant de recristallisation : cyclohexane
Rendement : 80%
Point de fusion : 114-115 °C

| Analyse élémentaire : | | | |
|---|---|---|---|
| Calculée<br>Trouvée | C : 67,52<br>C : 67,63 | H : 5,66<br>H : 5,51 | N : 6,06<br>N : 5,99 |

Caractéristiques spectrales :
Infrarouge :
1770 $cm^{-1}$ : ν CO (OCON)
1660 $cm^{-1}$ : ν CO (cétonique)
Résonance magnétique nucléaire (Solvant : DMSO $D_6$)
δ : 1,39 ppm, doublet, 3H, C-$CH_3$, J = 6,5Hz
δ : 3,35 ppm, singulet, 3H, N-$CH_3$

**EXEMPLE 22 : 3-METHYL 6-ISONICOTINOYL BENZOXAZOLINONE (CHLORHYDRATE)**

En procédant comme dans l'exemple 1, mais en remplaçant le chlorhydrate de chlorure d'acide nicotinique par le chlorhydrate de chlorure d'acide isonicotinique, on obtient le produit du titre.

**EXEMPLE 23 : 3-METHYL 6-(OUINOL-2-YL CARBONYL) BENZOXAZOLINONE**

En procédant comme dans l'exemple 1, mais en remplaçant le chlorhydrate de chlorure d'acide nicotinique par le chlorhydrate de chlorure d'acide 2-quinoléine carboxylique, on obtient le produit du titre.

**EXEMPLE 24 : 6-(OUINOL-3-YL CARBONYL) BENZOXAZOLINONE**

En procédant comme dans l'exemple 2, mais en remplaçant le chlorhydrate de chlorure d'acide nicotinique par le chlorhydrate de chlorure d'acide 3-quinoléine carboxylique, on obtient le produit du titre.

**EXEMPLE 25 : 3-METHYL 6-(INDOL- 3 YL CARBONYL) BENZOXAZOLINONE**

En procédant comme dans l'exemple 1, mais en remplaçant le chlorhydrate de chlorure d'acide nicotinique par le chlorure de l'acide 3-indole-carboxylique (préparé comme dans la demande de brevet belge BE 900425), on obtient le produit du titre.

**EXEMPLE 26 : 3-METHYL 6-(PYRROL-2-YL CARBONYL) BENZOXAZOLINONE**

En procédant comme dans l'exemple 1, mais en remplaçant le chlorhydrate de chlorure d'acide nicotinique par le chlorure de l'acide 2-pyrrole carboxylique, on obtient le produit du titre.

**EXEMPLE 27 : 6(ISOOUINOL-1-YL CARBONYL) BENZOXAZOLINONE**

En procédant comme dans l'exemple 2, mais en remplaçant le chlorhydrate de chlorure d'acide nicotinique par le chlorhydrate de chlorure d'acide 1-isoquinoléine carboxylique, on obtient le produit du titre.

**EXEMPLE 28 : 6-[(1-METHYL PYRROL-2YL)CARBONYL] BENZOXAZOLINONE**

En procédant comme dans l'exemple 2, mais en remplaçant le chlorhydrate de chlorure d'acide nicotinique par le chlorhydrate de chlorure de l'acide 1-méthyl-2-pyrrole carboxylique, on obtient le produit du titre.

**EXEMPLE 29 : 6-[(1-METHYL INDOL-2YL)CARBONYL] BENZOXAZOLINONE**

En procédant comme dans l'exemple 2, mais en remplaçant le chlorhydrate de chlorure d'acide nicotinique par le chlorure de l'acide 1-méthyl-2-indole carboxylique, on obtient le produit du titre.

**EXEMPLE 30 : 3-METHYL 6-[(5-METHOXY INDOL-2YL)CARBONYL] BENZOXAZOLINONE**

En procédant comme dans l'exemple 1, mais en remplaçant le chlorhydrate de chlorure d'acide nicotinique par le chlorure d'acide 5-méthoxy-2-indole carboxylique, on obtient le produit du titre.

**EXEMPLE 31 : 3-METHYL 6-[(5-CHLORO INDOL-2YL)CARBONYL] BENZOXAZOLINONE**

En procédant comme dans l'exemple 1, mais en remplaçant le chlorhydrate de chlorure d'acide nicotinique par le chlorure d'acide 5-chloro-2-indole carboxylique, on obtient le produit du titre.

**ETUDE PHARMACOLOGIOUE DES DERIVES DE L'INVENTION**

**EXEMPLE 32 : ETUDE DE LA TOXICITE AIGUE**

La toxicité aiguë a été appréciée après administration orale à des lots de 8 souris (26 ± 2 grammes) d'une dose de 650 $mg.kg^{-1}$. Les animaux ont été observés à intervalles réguliers au cours de la première journée et quotidiennement pendant les 2 semaines suivant le traitement.

Il apparaît que les composés de l'invention sont totalement atoxiques. Aucun décès n'est observé après administration d'une dose de 650 $mg.kg^{-1}$. On ne constate pas de troubles après administration de cette dose.

**EXEMPLE 33 : ETUDE DE L'ACTIVITE ANTIAGREGANTE PLAOUETTAIRE**

Un plasma riche en plaquettes est préparé à partir de sang humain citraté, provenant de donneurs n'ayant pris aucun médicament pendant les dix jours précédant le prélèvement.

L'agrégation des plaquettes dans ce milieu plasmatique est étudiée par turbidimétrie en employant l'acide arachidonique. Les produits de l'invention sont ajoutés au plasma trois minutes avant l'agoniste. Les produits manifestent une activité antagoniste de l'agrégation plaquettaire significative.

**EXEMPLE 34 : COMPOSITION PHARMACEUTIOUE : COMPRIMES**

Comprimés dosés à 50 mg de 6-nicotinoyl benzoxazolinone

Formule de préparation pour 1000 comprimés.

| | |
|---|---|
| 6-nicotinoyl benzoxazolinone . . . . | 50 g |
| Amidon de blé . . . . . . . . . . . | 15 g |
| Amidon de maïs . . . . . . . . . . . | 15 g |
| Lactose . . . . . . . . . . . . . . | 65 g |
| Stéarate de magnésium . . . . . . . . | 2 g |
| Silice . . . . . . . . . . . . . . . . | 1 g |
| Hydroxypropylcellulose . . . . . . . . | 2 g |

**Revendications**

**Revendications pour les Etats contractants suivants : AT, BE, CH, DE, DK, FR, GB, IT, LI, LU, NL, SE**

**1.** Dérivés de formule générale (I) :

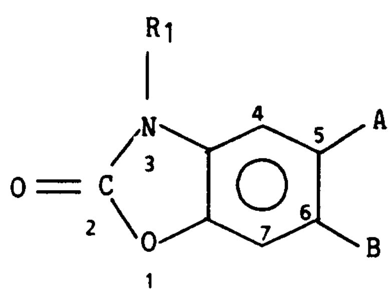

(I)

dans laquelle :

$R_1$ représente un atome d'hydrogène ou un groupement alkyle inférieur,

A représente un atome d'hydrogène et dans ce cas B représente un groupement CO-G, G étant :

- ou bien un groupement hétéroaryle choisi parmi furanne, indole, pyrrole, pyridine, quinoléïne, isoquinoléine, benzofuranne éventuellement substitué par un ou plusieurs groupements alkyle inférieur ou alkoxy inférieur, ou atome d'halogène,
- ou bien un groupement alkyle inférieur linéaire ou ramifié substitué par un groupement carboxyle,
- ou bien un groupement alcényle inférieur linéaire ou ramifié substitué par un groupement carboxyle,
- ou bien un groupement phényle ou naphtyle substitué par un groupement carboxyle,
- ou bien A forme avec B un groupement $CO(CH_2)_nCH(CH_3)$ avec n nombre entier égal à 1, 2, 3 ou 4 le groupement CO étant fixé au cycle aromatique de la benzoxazolinone en position 5 ou 6,

  ainsi que le cas échéant, leurs isomères et, lorsque le composé de formule (I) possède un groupement acide carboxylique, leurs sels d'addition à une base pharmaceutiquement acceptable et lorsque le composé de formule (I) comporte un groupement basique, leurs sels d'addition avec un acide pharmaceutiquement acceptable, étant entendu que alkyle inférieur, alkoxy inférieur et alcényle inférieur signifient des groupements linéaires ou ramifiés comprenant de 1 à 6 atomes de carbone.

2. Composés selon la revendication 1 pour lesquels B représente un groupement CO-G avec G hétéroaryle choisi parmi furanne, indole, pyrrole, pyridine, quinoléine, isoquinoléine, benzofuranne éventuellement substitué par un ou plusieurs groupements alkyle ou alkoxy inférieurs ou atome d'halogène ainsi que lorsque G comprend un groupement basique, leurs sels d'addition avec un acide pharmaceutiquement acceptable.

3. Composés selon la revendication 1 pour lesquels B représente un groupement CO-G avec G alkyle inférieur linéaire ou ramifié substitué par un groupement carboxyle, le cas échéant leurs isomères ainsi que leurs sels d'addition à une base pharmaceutiquement acceptable.

4. Composés selon la revendication 1 pour lesquels B représente un groupement CO-G avec G alcényle inférieur linéaire ou ramifié substitué par un groupement carboxyle, leurs isomères ainsi que leurs sels d'addition à une base pharmaceutiquement acceptable.

5. Composés selon la revendication 1 pour lesquels B représente un groupement phényle ou naphtyle substitué par un groupement carboxylique ainsi que leurs sels d'addition à une base pharmaceutiquement acceptable.

6. Composés selon la revendication 1 dans lesquels A forme avec B un groupement $CO(CH_2)_nCH(CH_3)$ avec n nombre entier égal à 1, 2, 3 ou 4, le groupement CO étant fixé au cycle aromatique de la benzoxazolinone en position 5 ou 6 ainsi que leurs isomères.

7. Composés selon l'une des revendications 1 ou 2 pour lesquels B représente un groupement CO-G avec G hétérocycle choisi parmi furanne ou pyridine ainsi que lorsque G représente la pyridine, leurs sels d'addition à un acide pharmaceutiquement acceptable.

8. Procédé de préparation des composés de formule (I) selon la revendication 1 caractérisé en ce que l'on utilise comme matière première un dérivé de formule (II) :

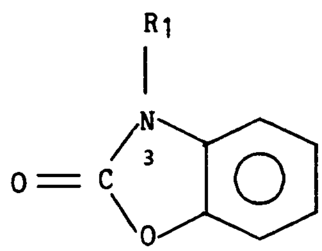

(II)

dans laquelle $R_1$ a la même définition que dans la formule (I),
que l'on traite en présence de diméthylformamide et de chlorure d'aluminium :

a) soit :

- par un chlorure d'acide de formule (III) :

**ClCOG (III)**

dans laquelle :
G a la même définition que dans la formule (I) à l'exception du cas où G représente un groupement alcényle inférieur substitué par un carboxyle,

- ou par un anhydride d'acide de formule (IV) :

**$O(OCG)_2$ (IV)**

dans laquelle G a la même définition que dans la formule (I),
pour obtenir un dérivé de formule (I/a) :

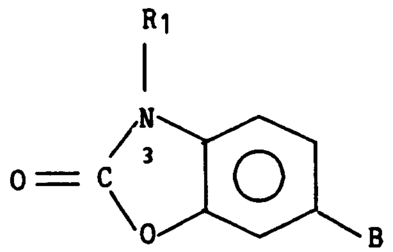

**(I/a)**

cas particulier des dérivés de formule (I) dans lequel A représente un atome d'hydrogène et B a la même signification que dans la formule (I) lorsque A représente un atome d'hydrogène,
dérivé de formule (I/a) que l'on purifie si nécessaire par une technique classique choisie parmi cristallisation ou chromatographie, dont on sépare le cas échéant les isomères et que l'on salifie si on le désire, lorsque B renferme un groupement acide carboxylique par une base pharmaceutiquement acceptable et lorsque B renferme un groupement basique par un acide pharmaceutiquement acceptable,
b) soit par un chlorure d'acide de formule (V) :

**$ClOC - (CH_2)_{n-1}CH= CH - CH_3$ (V)**

dans laquelle n a la même définition que dans la formule (I),
soit par un dérivé de formule :

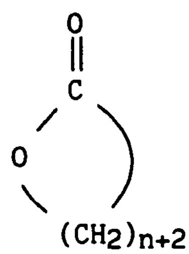

dans laquelle n a la même définition que dans la formule (I),
pour obtenir un mélange de dérivés de formule (I/b) :

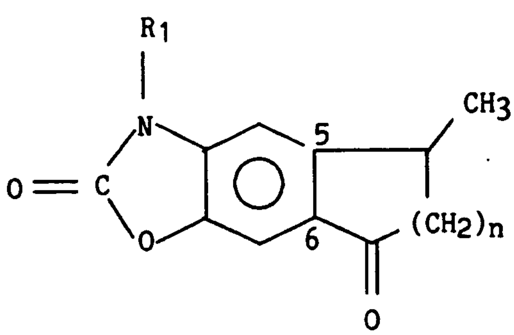

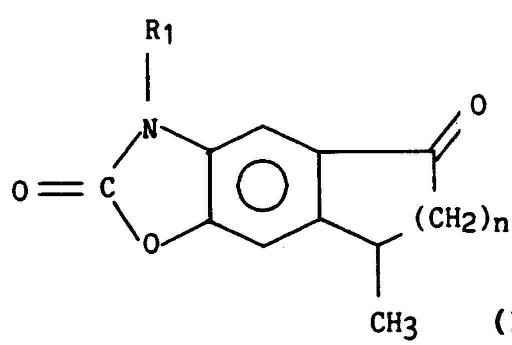

**(I/b)**

dans laquelle $R_1$ et n ont la même définition que dans la formule (I),
cas particulier de dérivés de formule (I) dans lesquels A forme avec B un groupement $CO(CH_2)_nCHCH_3$, dans laquelle n à la même définition que dans la formule (I), le groupement CO étant fixé soit en position 5, soit en position 6 de la benzoxazolinone, dérivés de formule (I/b) que l'on sépare et purifie par une ou plusieurs techniques choisies parmi cristallisation ou chromatographie.

**9.** Procédé de synthèse des composés de formule (I/c) selon la revendication 1 :

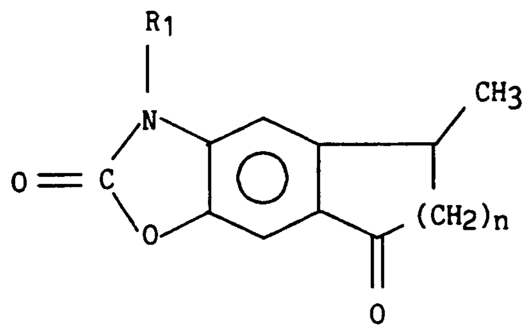

(I/c)

dans lesquels $R_1$ et n ont la même définition que dans la formule (I)caractérisé en ce que l'on soumet un dérivé de formule (IV) :

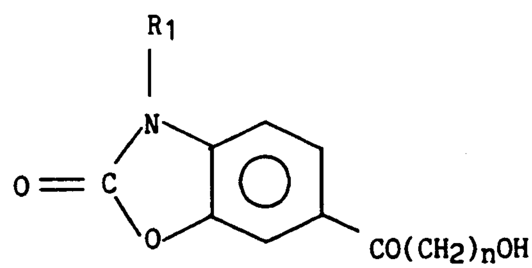

(IV)

dans lequel $R_1$ et n ont la même définition que dans la formule (I),
à l'action du chlorure d'aluminium en présence de diméthylformamide,
pour obtenir un dérivé de formule (I/c) que l'on purifie si on le désire, par une ou plusieurs techniques choisies parmi cristallisation et/ou chromatographie.

**10.** Composition pharmaceutique contenant comme principe actif au moins un composé selon l'une des revendications 1 à 7 en combinaison avec un ou plusieurs excipients ou véhicules inertes, non-toxiques, pharmaceutiquement acceptables.

**11.** Composition pharmaceutique selon la revendication 10 contenant au moins un principe actif selon l'une des revendications 1 à 7 utilisables dans le traitement et la prévention des accidents ischémiques artériels périphériques et cérébrovasculaires.

**Revendications pour l'Etat contractant suivant : ES**

**1.** Procédé de préparation de dérivés de formule générale (I) :

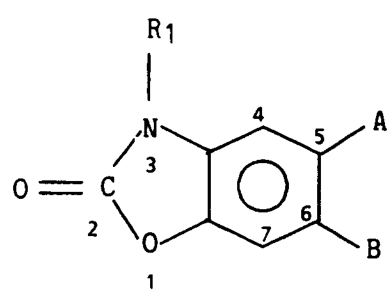

(I)

dans laquelle :

$R_1$ représente un atome d'hydrogène ou un groupement alkyle inférieur,

A représente un atome d'hydrogène et dans ce cas B représente un groupement CO-G, G étant

:

- ou bien un groupement hétéroaryle choisi parmi furanne, indole, pyrrole, pyridine, quinoléïne, isoquinoléine, benzofuranne éventuellement substitué par un ou plusieurs groupements alkyle inférieur ou alkoxy inférieur, ou atome d'halogène,
- ou bien un groupement alkyle inférieur linéaire ou ramifié substitué par un groupement carboxyle,
- ou bien un groupement alcényle inférieur linéaire ou ramifié substitué par un groupement carboxyle,
- ou bien un groupement phényle ou naphtyle substitué par un groupement carboxyle,
- ou bien A forme avec B un groupement $CO(CH_2)_nCH(CH_3)$ avec n nombre entier égal à 1, 2, 3 ou 4 le groupement CO étant fixé au cycle aromatique de la benzoxazolinone en position 5 ou 6,

ainsi que le cas échéant, leurs isomères et, lorsque le composé de formule (I) possède un groupement acide carboxylique, leurs sels d'addition à une base pharmaceutiquement acceptable et lorsque le composé de formule (I) comporte un groupement basique, leurs sels d'addition avec un acide pharmaceutiquement acceptable, étant entendu que alkyle inférieur, alkoxy inférieur et alcényle inférieur signifient des groupements linéaires ou ramifiés comprenant de 1 à 6 atomes de carbone,

caractérisé en ce que l'on utilise comme matière première un dérivé de formule (II) :

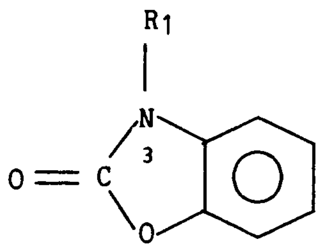

(II)

dans laquelle $R_1$ a la même définition que dans la formule (I),
que l'on traite en présence de diméthylformamide et de chlorure d'aluminium :

a) soit :

- par un chlorure d'acide de formule (III) :

**ClCOG (III)**

dans laquelle :
G a la même définition que dans la formule (I) à l'exception du cas où G représente un groupement alcényle inférieur substitué par un carboxyle,

- ou par un anhydride d'acide de formule (IV) :

**$O(OCG)_2$ (IV)**

dans laquelle G a la même définition que dans la formule (I),
pour obtenir un dérivé de formule (I/a) :

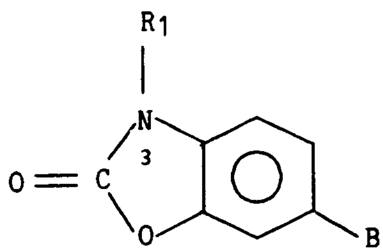

(I/a)

cas particulier des dérivés de formule (I) dans lequel A représente un atome d'hydrogène et B a la

même signification que dans la formule (I) lorsque A représente un atome d'hydrogène, dérivé de formule (I/a) que l'on purifie si nécessaire par une technique classique choisie parmi cristallisation ou chromatographie, dont on sépare le cas échéant les isomères et que l'on salifie si on le désire, lorsque B renferme un groupement acide carboxylique par une base pharmaceutiquement acceptable et lorsque B renferme un groupement basique par un acide pharmaceutiquement acceptable,

b) soit par un chlorure d'acide de formule (V) :

**$ClOC-(CH_2)_{n-1}CH=CH-CH_3$ (V)**

dans laquelle n a la même définition que dans la formule (I),

soit par un dérivé de formule :

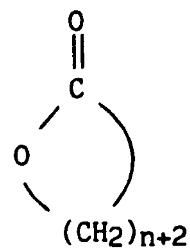

dans laquelle n a la même définition que dans la formule (I), pour obtenir un mélange de dérivés de formule (I/b) :

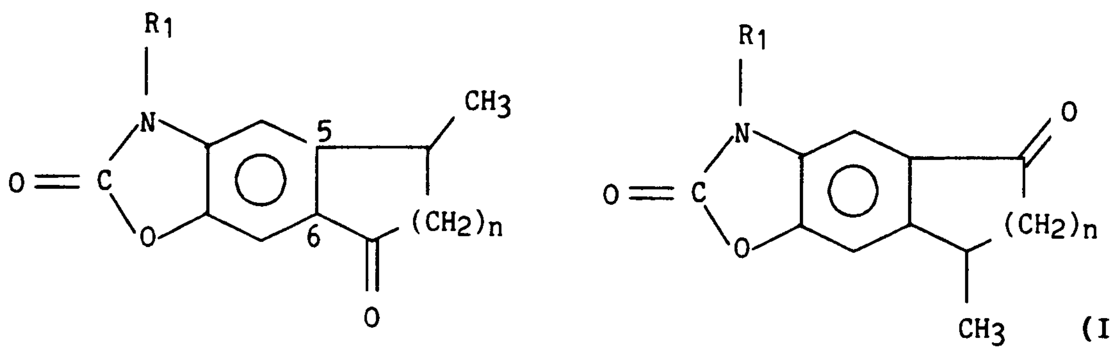

(I/b)

dans laquelle $R_1$ et n ont la même définition que dans la formule (I), cas particulier de dérivés de formule (I) dans lesquels A forme avec B un groupement $CO(CH_2)_nCHCH_3$, dans laquelle n à la même définition que dans la formule (I), le groupement CO étant fixé soit en position 5, soit en position 6 de la benzoxazolinone, dérivés de formule (I/b) que l'on sépare et purifie par une ou plusieurs techniques choisies parmi cristallisation ou chromatographie.

**2.** Procédé de synthèse des composés de formule (I/c) :

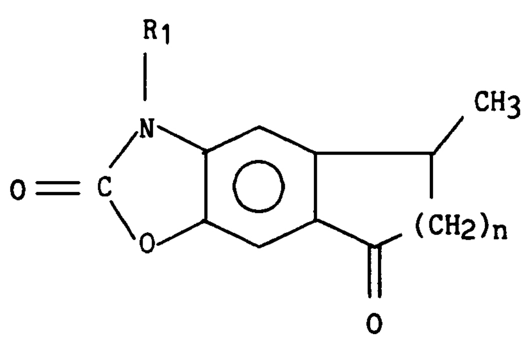

(I/c)

dans lesquels $R_1$ et n ont la même définition que dans la formule (I) de la revendication 1 caractérisé en ce que l'on soumet un dérivé de formule (IV) :

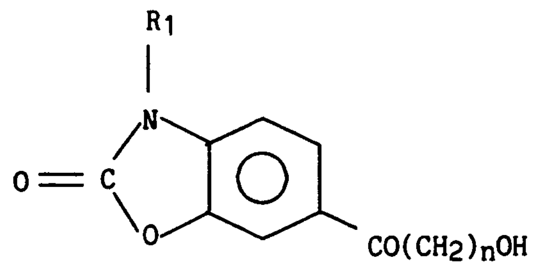

(IV)

dans lequel $R_1$ et n ont la même définition que dans la formule (I),
à l'action du chlorure d'aluminium en présence de diméthylformamide,
pour obtenir un dérivé de formule (I/c) que l'on purifie si on le désire, par une ou plusieurs techniques choisies parmi cristallisation et/ou chromatographie.

**3.** Procédé selon la revendication 1 de préparation de composés de formule (I) pour lesquels B représente un groupement CO-G avec G hétéroaryle choisi parmi furanne, indole, pyrrole, pyridine, quinoléine, isoquinoléine, benzofuranne éventuellement substitué par un ou plusieurs groupements alkyle ou alkoxy inférieurs ou atome d'halogène ainsi que lorsque G comprend un groupement basique, leurs sels d'addition avec un acide pharmaceutiquement acceptable.

**4.** Procédé selon la revendication 1 de préparation de composés de formule (I) pour lesquels B représente un groupement CO-G avec G alkyle inférieur linéaire ou ramifié substitué par un groupement carboxyle, le cas échéant leurs isomères ainsi que leurs sels d'addition à une base pharmaceutiquement acceptable.

**5.** Procédé selon la revendication 1 de préparation de composés de formule (I) pour lesquels B représente un groupement CO-G avec G alcényle inférieur linéaire ou ramifié substitué par un groupement carboxyle, leurs isomères ainsi que leurs sels d'addition à une base pharmaceutiquement acceptable.

**6.** Procédé selon la revendication 1 de préparation de composés de formule (I) pour lesquels B représente un groupement phényle ou naphtyle substitué par un groupement carboxylique ainsi que leurs sels d'addition à une base pharmaceutiquement acceptable.

**7.** Procédé selon l'une des revendications 1 ou 2 de préparation de composés de formule (I) pour lesquels A forme avec B un groupement $CO(CH_2)_nCH(CH_3)$ avec n nombre entier égal à 1, 2, 3 ou 4, le groupement CO étant fixé au cycle aromatique de la benzoxazolinone en position 5 ou 6 ainsi que leurs isomères.

**8.** Procédé selon la revendication 1 de préparation des composés de formule (I) pour lesquels B représente un groupement CO-G avec G hétérocycle choisi parmi furanne ou pyridine ainsi que lorsque G représente la pyridine, leurs sels d'addition à un acide pharmaceutiquement acceptable.

**9.** Procédé de préparation de compositions pharmaceutiques contenant un composé obtenu selon la revendication 1, leurs isomères ou leurs sels d'addition à un acide pharmaceutiquement acceptable.

**10.** Procédé selon la revendication 9 de préparation de compositions pharmaceutiques utile dans le traitement ou la prévention des accidents ischémiques artériels périphériques et cérébro vasculaires.

**Revendications pour l'Etat contractant suivant : GR**

**1.** Procédé de préparation de dérivés de formule générale (I) :

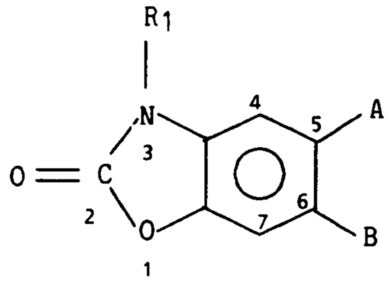

(I)

dans laquelle :

$R_1$ représente un atome d'hydrogène ou un groupement alkyle inférieur,

A représente un atome d'hydrogène et dans ce cas B représente un groupement CO-G, G étant :

- ou bien un groupement hétéroaryle choisi parmi furanne, indole, pyrrole, pyridine, quinoléïne, isoquinoléine, benzofuranne éventuellement substitué par un ou plusieurs groupements alkyle inférieur ou alkoxy inférieur, ou atome d'halogène,
- ou bien un groupement alkyle inférieur linéaire ou ramifié substitué par un groupement carboxyle,
- ou bien un groupement alcényle inférieur linéaire ou ramifié substitué par un groupement carboxyle,
- ou bien un groupement phényle ou naphtyle substitué par un groupement carboxyle,
- ou bien A forme avec B un groupement $CO(CH_2)_nCH(CH_3)$ avec n nombre entier égal à 1, 2, 3 ou 4 le groupement CO étant fixé au cycle aromatique de la benzoxazolinone en position 5 ou 6,

  ainsi que le cas échéant, leurs isomères et, lorsque le composé de formule (I) possède un groupement acide carboxylique, leurs sels d'addition à une base pharmaceutiquement acceptable et lorsque le composé de formule (I) comporte un groupement basique, leurs sels d'addition avec un acide pharmaceutiquement acceptable, étant entendu que alkyle inférieur, alkoxy inférieur et alcényle inférieur signifient des groupements linéaires ou ramifiés comprenant de 1 à 6 atomes de carbone,

caractérisé en ce que l'on utilise comme matière première un dérivé de formule (II) :

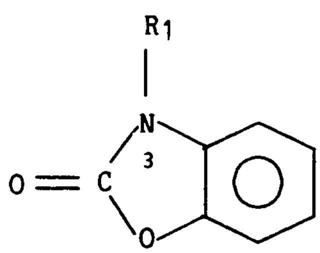

(II)

dans laquelle $R_1$ a la même définition que dans la formule (I),
que l'on traite en présence de diméthylformamide et de chlorure d'aluminium :

a) soit :

- par un chlorure d'acide de formule (III) :

**ClCOG (III)**

dans laquelle :
G a la même définition que dans la formule (I) à l'exception du cas où G représente un groupement alcényle inférieur substitué par un carboxyle,

- ou par un anhydride d'acide de formule (IV) :

**$O(OCG)_2$ (IV)**

dans laquelle G a la même définition que dans la formule (I),
pour obtenir un dérivé de formule (I/a) :

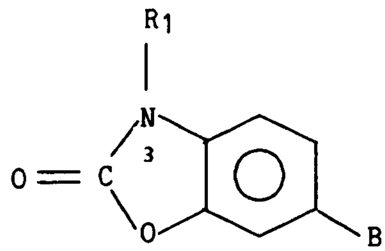

(I/a)

cas particulier des dérivés de formule (I) dans lequel A représente un atome d'hydrogène et B a la même signification que dans la formule (I) lorsque A représente un atome d'hydrogène,
dérivé de formule (I/a) que l'on purifie si nécessaire par une technique classique choisie parmi cristallisation ou chromatographie, dont on sépare le cas échéant les isomères et que l'on salifie si on le désire, lorsque B renferme un groupement acide carboxylique par une base pharmaceutiquement acceptable et lorsque B renferme un groupement basique par un acide pharmaceutiquement acceptable,
b) soit par un chlorure d'acide de formule (V) :

**$ClOC - (CH_2)_{n-1}CH= CH - CH_3$ (V)**

dans laquelle n a la même définition que dans la formule (I),
soit par un dérivé de formule :

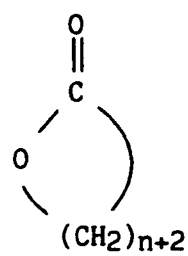

dans laquelle n a la même définition que dans la formule (I),
pour obtenir un mélange de dérivés de formule (I/b) :

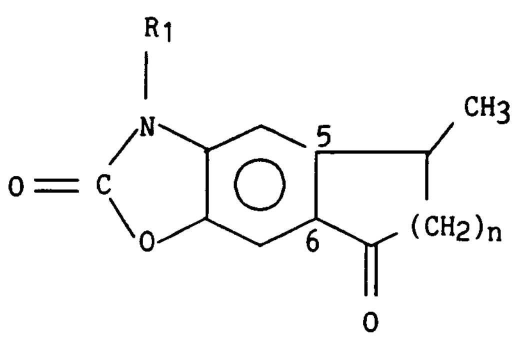

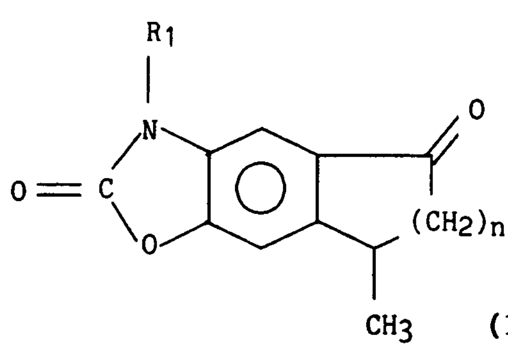

(I/b)

dans laquelle $R_1$ et n ont la même définition que dans la formule (I),

cas particulier de dérivés de formule (I) dans lesquels A forme avec B un groupement $CO(CH_2)_nCHCH_3$, dans laquelle n à la même définition que dans la formule (I), le groupement CO étant fixé soit en position 5, soit en position 6 de la benzoxazolinone, dérivés de formule (I/b) que l'on sépare et purifie par une ou plusieurs techniques choisies parmi cristallisation ou chromatographie.

**2.** Procédé de synthèse des composés de formule (I/c) :

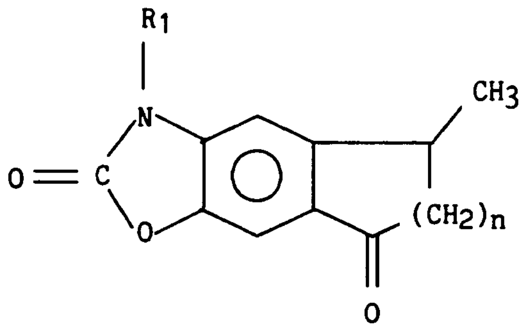

(I/c)

dans lesquels $R_1$ et n ont la même définition que dans la formule (I) de la revendication 1 caractérisé en ce que l'on soumet un dérivé de formule (IV) :

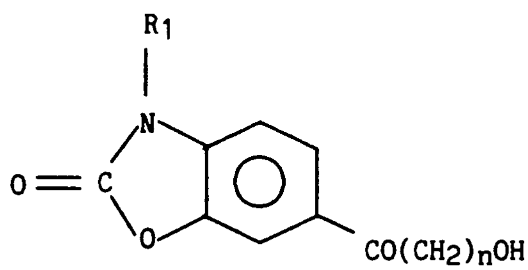

(IV)

dans lequel $R_1$ et n ont la même définition que dans la formule (I),
à l'action du chlorure d'aluminium en présence de diméthylformamide, pour obtenir un dérivé de formule (I/c) que l'on purifie si on le désire, par une ou plusieurs techniques choisies parmi cristallisation et/ou chromatographie.

**3.** Procédé selon la revendication 1 de préparation de composés de formule (I) pour lesquels B représente un groupement CO-G avec G hétéroaryle choisi parmi furanne, indole, pyrrole, pyridine, quinoléine, isoquinoléine, benzofuranne éventuellement substitué par un ou plusieurs groupements alkyle ou alkoxy inférieurs ou atome d'halogène ainsi que lorsque G comprend un groupement basique, leurs sels d'addition avec un acide pharmaceutiquement acceptable.

**4.** Procédé selon la revendication 1 de préparation de composés de formule (I) pour lesquels B représente un groupement CO-G avec G alkyle inférieur linéaire ou ramifié substitué par un groupement carboxyle, le cas échéant leurs isomères ainsi que leurs sels d'addition à une base pharmaceutiquement acceptable.

**5.** Procédé selon la revendication 1 de préparation de composés de formule (I) pour lesquels B représente un groupement CO-G avec G alcényle inférieur linéaire ou ramifié substitué par un groupement carboxyle, leurs isomères ainsi que leurs sels d'addition à une base pharmaceutiquement acceptable.

**6.** Procédé selon la revendication 1 de préparation de composés de formule (I) pour lesquels B représente un groupement phényle ou naphtyle substitué par un groupement carboxylique ainsi que leurs sels d'addition à une base pharmaceutiquement acceptable.

7. Procédé selon l'une des revendications 1 ou 2 de préparation de composés de formule (I) pour lesquels A forme avec B un groupement $CO(CH_2)_nCH(CH_3)$ avec n nombre entier égal à 1, 2, 3 ou 4, le groupement CO étant fixé au cycle aromatique de la benzoxazolinone en position 5 ou 6 ainsi que leurs isomères.

8. Procédé selon la revendication 1 de préparation des composés de formule (I) pour lesquels B représente un groupement CO-G avec G hétérocycle choisi parmi furanne ou pyridine ainsi que lorsque G représente la pyridine, leurs sels d'addition à un acide pharmaceutiquement acceptable.

9. Procédé de préparation de compositions pharmaceutiques contenant un composé obtenu selon la revendication 1, leurs isomères ou leurs sels d'addition à un acide pharmaceutiquement acceptable.

10. Procédé selon la revendication 9 de préparation de compositions pharmaceutiques utile dans le traitement ou la prévention des accidents ischémiques artériels périphériques et cérébro vasculaires.

**Claims**

**Claims for the following Contracting States : AT, BE, CH, DE, DK, FR, GB, IT, LI, LU, NL, SE**

1. Compounds of the general formula (I) :

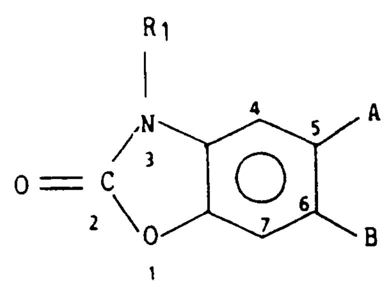

(I)

wherein :

$R_1$ represents a hydrogen atom or a lower alkyl group,

A represents a hydrogen atom and, in that case, B represents a CO-G group, G representing :

- a heteroaryl group selected from furan, indole, pyrrole, pyridine, quinoline, isoquinoline and benzofuran each optionally substituted by one or more lower alkyl or lower alkoxy groups, or halogen,
- a straight-chain or branched lower alkyl group substituted by a carboxy group,
- a straight-chain or branched lower alkenyl group substituted by a carboxy group,
- or a phenyl or naphthyl group substituted by a carboxy group,
- or A and B together form a $CO(CH_2)_nCH(CH_3)$ group in which n is an integer 1, 2, 3 or 4, the CO group being bonded to the aromatic ring of the benzoxazolinone at the 5 or 6 position,

and also, where applicable, the isomers thereof and, when the compound of formula (I) contains a carboxylic acid group, the addition salts thereof with a pharmaceutically acceptable base and, when the compound of formula (I) contains a basic group, the addition salts thereof with a pharmaceutically acceptable acid, it being understood that lower alkyl, lower alkoxy and lower alkenyl denote straight-chain or branched groups containing from 1 to 6 carbon atoms.

2. Compounds according to claim 1 in which B represents a CO-G group in which G represents heteroaryl selected from furan, indole, pyrrole, pyridine, quinoline, isoquinoline and benzofuran each optionally substituted by one or more lower alkyl or lower alkoxy groups or halogen, and also, when G contains a basic group, the addition salts thereof with a pharmaceutically acceptable acid.

3. Compounds according to claim 1 in which B represents a CO-G group in which G represents straight-chain or branched lower alkyl substituted by a carboxy group, where applicable the isomers thereof, and also the addition salts thereof with a pharmaceutically acceptable base.

**4.** Compounds according to claim 1 in which B represents a CO-G group in which G represents straight-chain or branched lower alkenyl substituted by a carboxy group, the isomers thereof, and also the addition salts thereof with a pharmaceutically acceptable base.

**5.** Compounds according to claim 1 in which B represents a phenyl or naphthyl group substituted by a carboxylic group, and also the addition salts thereof with a pharmaceutically acceptable base.

**6.** Compounds according to claim 1 in which A and B together form a $CO(CH_2)_nCH(CH_3)$ group in which n is an integer 1, 2, 3 or 4, the CO group being bonded to the aromatic ring of the benzoxazolinone at the 5 or 6 position, and also the isomers thereof.

**7.** Compounds according to either claim 1 or 2 in which B represents a CO-G group in which G represents a heterocycle selected from furan and pyridine, and also, when G represents pyridine, the addition salts thereof with a pharmaceutically acceptable acid.

**8.** Process for the preparation of compounds of formula (I) according to claim 1, characterised in that there is used as starting material a compound of formula (II) :

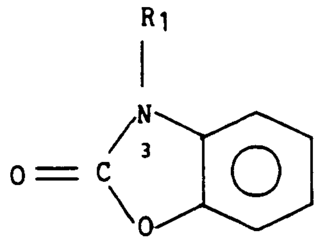

(II)

in which $R_1$ is as defined for formula (I),
which is treated in the presence of dimethylformamide and aluminium chloride :

a) either :

- with an acid chloride of formula (III) :

**ClCOG** (III)

in which :
G is as defined for formula (I) with the exception of the case in which G represents a lower alkenyl group substituted by a carboxy group,

- or with an acid anhydride of formula (IV) :

**$O(OCG)_2$** (IV)

in which G is as defined for formula (I),
to obtain a compound of formula (I/a) :

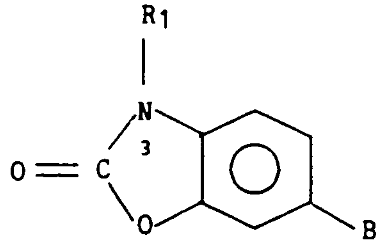

(I/a)

a particular instance of compounds of formula (I) in which A represents a hydrogen atom and B is as defined for formula (I) when A represents a hydrogen atom,
which compound of formula (I/a) is purified, if necessary, by a conventional method selected from

crystallisation and chromatography, is separated, where applicable, into its isomers and, if desired, when B contains a carboxylic acid group is converted into a salt with a pharmaceutically acceptable base and, when B contains a basic group is converted into a salt with a pharmaceutically acceptable acid,

b) or

with an acid chloride or formula (V) :

**$ClOC-(CH_2)_{n-1}CH=CH-CH_3$** (V)

in which n is as defined for formula (I),

or with a compound of formula :

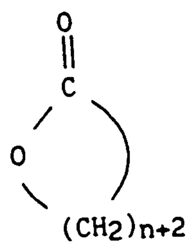

in which n is as defined for formula (I), to obtain a mixture of compounds of formula (I/b) :

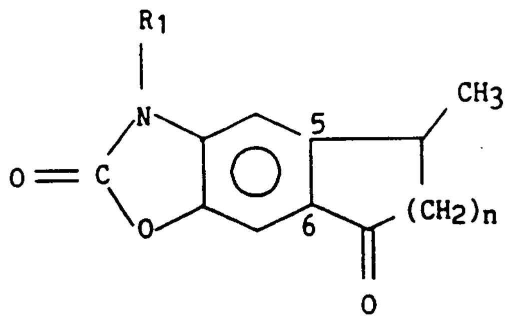

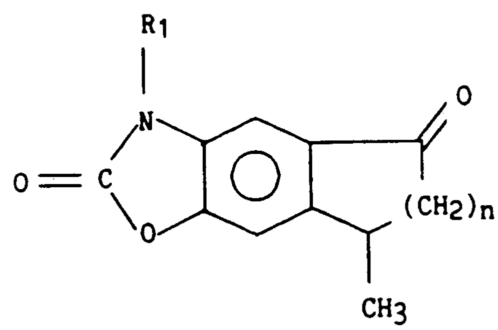

(I/b)

in which $R_1$ and n are as defined for formula (I),

a particular instance of compounds of formula (I) in which A and B together form a $CO(CH_2)_nCHCH_3$ group in which n is as defined for formula (I), the group CO being bonded either at the 5 position, or at the 6 position, of the benzoxazolinone, which compounds of formula (I/b) are separated and purified by one or more methods selected from crystallisation and chromatography.

**9.** Process for the synthesis of compounds of formula (I/c) according to claim 1 :

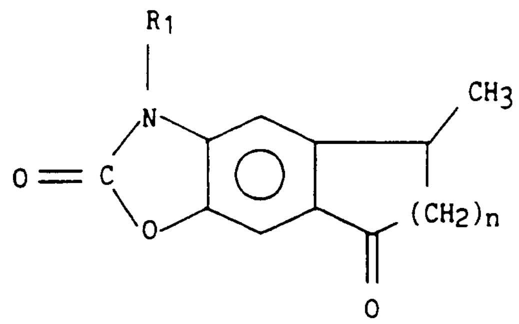

(I/c)

in which $R_1$ and n are as defined for formula (I),
characterised in that a compound of formula (IV) :

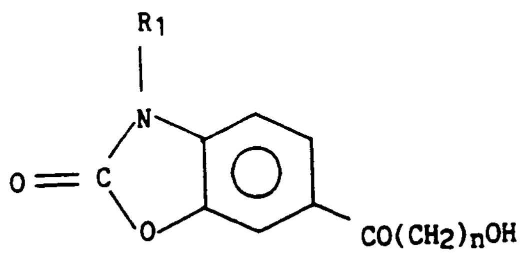

(IV)

in which $R_1$ and n are as defined for formula (I),
is subjected to the action of aluminium chloride in the presence of dimethylformamide
to obtain a compound of formula (I/c), which is purified, if desired, by one or more methods selected from crystallisation and/or chromatography.

**10.** Pharmaceutical composition comprising as active ingredient at least one compound according to any one of claims 1 to 7 in combination with one or more pharmaceutically acceptable, inert, non-toxic excipients or carriers.

**11.** Pharmaceutical composition according to claim 10 comprising at least one active ingredient according to any one of claims 1 to 7, which can be used in the treatment and prevention of cerebrovascular and peripheral arterial ischaemic accidents.

**Claims for the following Contracting State : ES**

**1.** Process for the preparation of compounds of the general formula (I) :

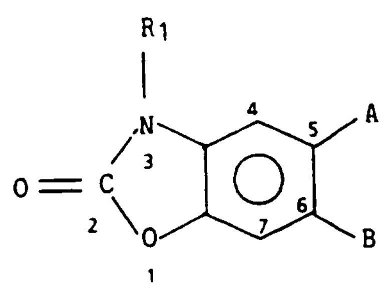

(I)

wherein :

$R_1$ represents a hydrogen atom or a lower alkyl group,

A represents a hydrogen atom and, in that case, B represents a CO-G group, G representing :

- a heteroaryl group selected from furan, indole, pyrrole, pyridine, quinoline, isoquinoline and benzofuran each optionally substituted by one or more lower alkyl or lower alkoxy groups, or halogen,
- a straight-chain or branched lower alkyl group substituted by a carboxy group,
- a straight-chain or branched lower alkenyl group substituted by a carboxy group,
- or a phenyl or naphthyl group substituted by a carboxy group,
- or A and B together form a $CO(CH_2)_nCH(CH_3)$ group in which n is an integer 1, 2, 3 or 4, the CO group being bonded to the aromatic ring of the benzoxazolinone at the 5 or 6 position,

and also, where applicable, the isomers thereof and, when the compound of formula (I) contains a carboxylic acid group, the addition salts thereof with a pharmaceutically acceptable base and, when the compound of formula (I) contains a basic group, the addition salts thereof with a pharmaceutically acceptable acid, it being understood that lower alkyl, lower alkoxy and lower alkenyl denote straight-chain or branched groups containing from 1 to 6 carbon atoms,

characterised in that there is used as starting material a compound of formula (II) :

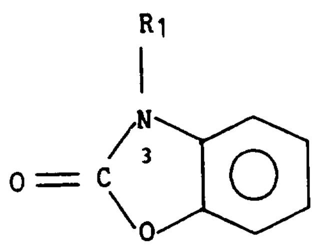

(II)

in which $R_1$ is as defined for formula (I),
which is treated in the presence of dimethylformamide and aluminium chloride :

a) either :

- with an acid chloride of formula (III) :

**ClCOG** (III)

in which :
G is as defined for formula (I) with the exception of the case in which G represents a lower alkenyl group substituted by a carboxy group,

- or with an acid anhydride of formula (IV) :

**$O(OCG)_2$** (IV)

in which G is as defined for formula (I),
to obtain a compound of formula (I/a) :

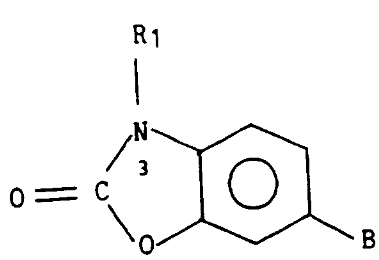

(I/a)

a particular instance of compounds of formula (I) in which A represents a hydrogen atom and B is as defined for formula (I) when A represents a hydrogen atom,
which compound of formula (I/a) is purified, if necessary, by a conventional method selected from crystallisation and chromatography, is separated, where applicable, into its isomers and, if desired,

when B contains a carboxylic acid group is converted into a salt with a pharmaceutically acceptable base and, when B contains a basic group is converted into a salt with a pharmaceutically acceptable acid,

b) or

with an acid chloride or formula (V) :

$ClOC - (CH_2)_{n-1}CH{=}CH{-}CH_3$ (V)

in which n is as defined for formula (I),

or with a compound of formula :

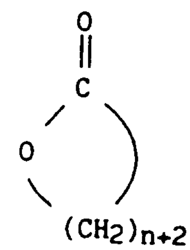

in which n is as defined for formula (I), to obtain a mixture of compounds of formula (I/b) :

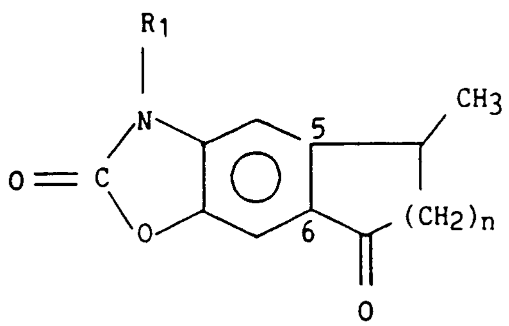

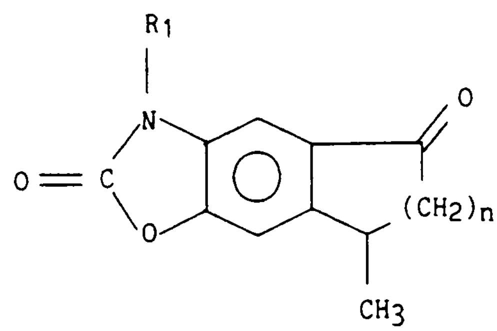

(I/b)

in which $R_1$ and n are as defined for formula (I),

a particular instance of compounds of formula (I) in which A and B together form a $CO(CH_2)_nCHCH_3$ group in which n is as defined for formula (I), the group CO being bonded either at the 5 position, or at the 6 position, of the benzoxazolinone, which compounds of formula (I/b) are separated and purified by one or more methods selected from crystallisation and chromatography.

**2.** Process for the synthesis of compounds of formula (I/c) :

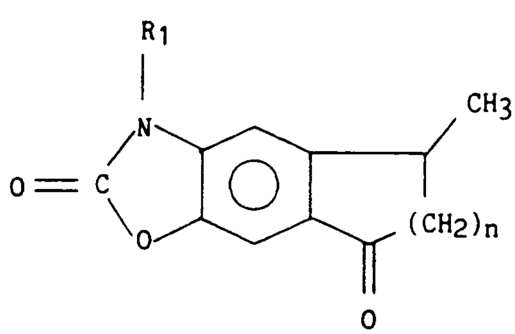

(I/c)

in which $R_1$ and n are as defined for formula (I) of claim 1, characterised in that a compound of formula (IV) :

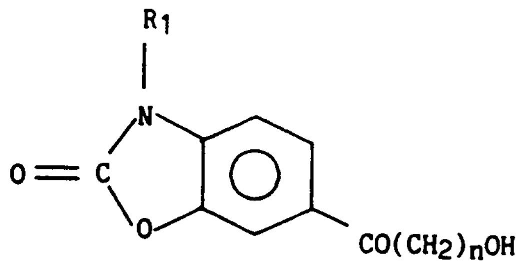

(IV)

in which $R_1$ and n are as defined for formula (I),
is subjected to the action of aluminium chloride in the presence of dimethylformamide
to obtain a compound of formula (I/c), which is purified, if desired, by one or more methods selected from crystallisation and/or chromatography.

**3.** Process according to claim 1 for the preparation of compounds of formula (I) in which B represents a CO-G group in which G represents heteroaryl selected from furan, indole, pyrrole, pyridine, quinoline, isoquinoline and benzofuran each optionally substituted by one or more lower alkyl or lower alkoxy groups or halogen, and also, when G contains a basic group, the addition salts thereof with a pharmaceutically acceptable acid.

**4.** Process according to claim 1 for the preparation of compounds of formula (I) in which B represents a CO-G group in which G represents straight-chain or branched lower alkyl substituted by a carboxy group, where applicable the isomers thereof, and also the addition salts thereof with a pharmaceutically acceptable base.

**5.** Process according to claim 1 for the preparation of compounds of formula (I) in which B represents a CO-G group in which G represents straight-chain or branched lower alkenyl substituted by a carboxy group, the isomers thereof, and also the addition salts thereof with a pharmaceutically acceptable base.

**6.** Process according to claim 1 for the preparation of compounds of formula (I) in which B represents a phenyl or naphthyl group substituted by a carboxylic group, and also the addition salts thereof with a pharmaceutically acceptable base.

**7.** Process according to either claim 1 or 2 for the preparation of compounds of formula (I) in which A and B together form a $CO(CH_2)_nCH(CH_3)$ group in which n is an integer 1, 2, 3 or 4, the CO group being bonded to the aromatic ring of the benzoxazolinone at the 5 or 6 position, and also the isomers thereof.

**8.** Process according to claim 1 for the preparation of compounds of formula (I) in which B represents a CO-G group in which G represents a heterocycle selected from furan and pyridine, and also, when G represents pyridine, the addition salts thereof with a pharmaceutically acceptable acid.

**9.** Process for the preparation of pharmaceutical compositions comprising a compound obtained in accordance with claim 1, an isomer thereof or an addition salt thereof with a pharmaceutically acceptable acid.

**10.** Process according to claim 9 for the preparation of pharmaceutical compositions useful in the treatment or prevention of cerebrovascular and peripheral arterial ischaemic accidents.

**Claims for the following Contracting State : GR**

**1.** Process for the preparation of compounds of the general formula (I) :

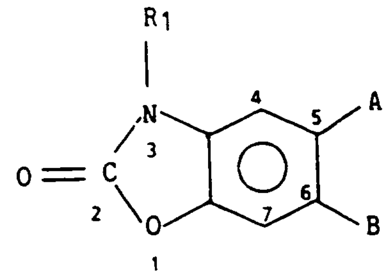

(I)

wherein :

$R_1$ represents a hydrogen atom or a lower alkyl group,

A represents a hydrogen atom and, in that case, B represents a CO-G group, G representing :

- a heteroaryl group selected from furan, indole, pyrrole, pyridine, quinoline, isoquinoline and benzofuran each optionally substituted by one or more lower alkyl or lower alkoxy groups, or halogen,
- a straight-chain or branched lower alkyl group substituted by a carboxy group,
- a straight-chain or branched lower alkenyl group substituted by a carboxy group,
- or a phenyl or naphthyl group substituted by a carboxy group,
- or A and B together form a $CO(CH_2)_nCH(CH_3)$ group in which n is an integer 1, 2, 3 or 4, the CO group being bonded to the aromatic ring of the benzoxazolinone at the 5 or 6 position,

and also, where applicable, the isomers thereof and, when the compound of formula (I) contains a carboxylic acid group, the addition salts thereof with a pharmaceutically acceptable base and, when the compound of formula (I) contains a basic group, the addition salts thereof with a pharmaceutically acceptable acid, it being understood that lower alkyl, lower alkoxy and lower alkenyl denote straight-chain or branched groups containing from 1 to 6 carbon atoms,

characterised in that there is used as starting material a compound of formula (II) :

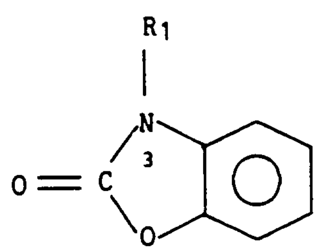

(II)

in which $R_1$ is as defined for formula (I),

which is treated in the presence of dimethylformamide and aluminium chloride :

a) either :

- with an acid chloride of formula (III) :

**ClCOG** (III)

in which :

G is as defined for formula (I) with the exception of the case in which G represents a lower alkenyl group substituted by a carboxy group,

- or with an acid anhydride of formula (IV) :

**$O(OCG)_2$** (IV)

in which G is as defined for formula (I), to obtain a compound of formula (I/a) :

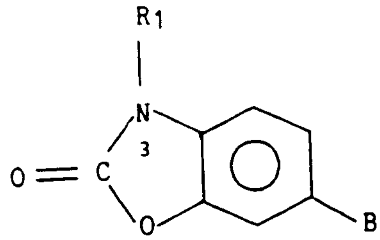

(I/a)

a particular instance of compounds of formula (I) in which A represents a hydrogen atom and B is as defined for formula (I) when A represents a hydrogen atom,
which compound of formula (I/a) is purified, if necessary, by a conventional method selected from crystallisation and chromatography, is separated, where applicable, into its isomers and, if desired, when B contains a carboxylic acid group is converted into a salt with a pharmaceutically acceptable base and, when B contains a basic group is converted into a salt with a pharmaceutically acceptable acid,
b) or
with an acid chloride or formula (V) :

**$ClOC - (CH_2)_{n-1}CH=CH-CH_3$** (V)

in which n is as defined for formula (I),
or with a compound of formula :

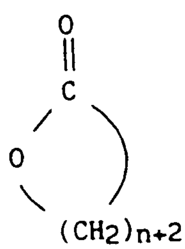

in which n is as defined for formula (I), to obtain a mixture of compounds of formula (I/b) :

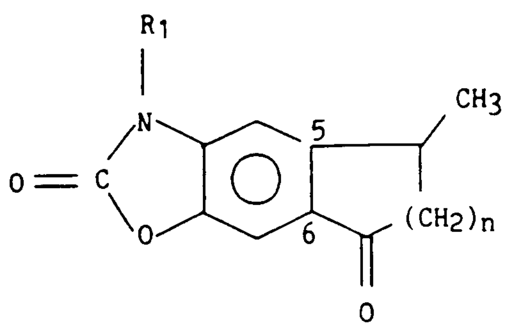

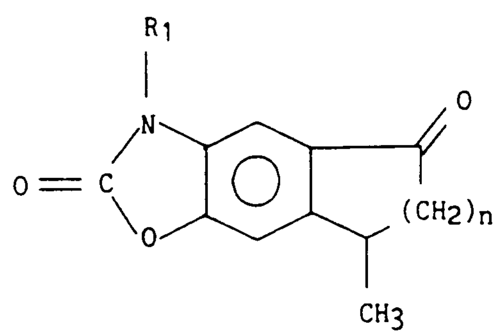

(I/b)

in which $R_1$ and n are as defined for formula (I),
a particular instance of compounds of formula (I) in which A and B together form a $CO(CH_2)_nCHCH_3$

group in which n is as defined for formula (I), the group CO being bonded either at the 5 position, or at the 6 position, of the benzoxazolinone, which compounds of formula (I/b) are separated and purified by one or more methods selected from crystallisation and chromatography.

**2.** Process for the synthesis of compounds of formula (I/c) :

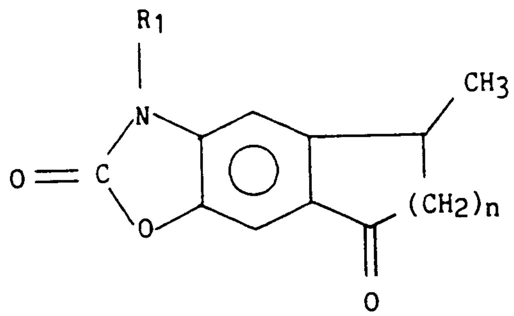

(I/c)

in which $R_1$ and n are as defined for formula (I) of claim 1, characterised in that a compound of formula (IV) :

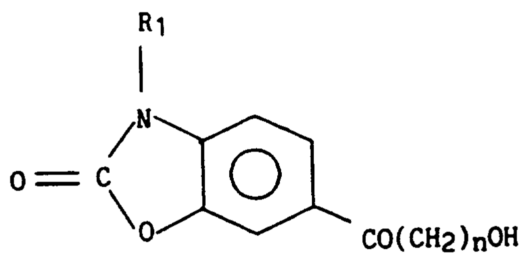

(IV)

in which $R_1$ and n are as defined for formula (I),
is subjected to the action of aluminium chloride in the presence of dimethylformamide
to obtain a compound of formula (I/c), which is purified, if desired, by one or more methods selected from crystallisation and/or chromatography.

**3.** Process according to claim 1 for the preparation of compounds of formula (I) in which B represents a CO-G group in which G represents heteroaryl selected from furan, indole, pyrrole, pyridine, quinoline, isoquinoline and benzofuran each optionally substituted by one or more lower alkyl or lower alkoxy groups or halogen, and also, when G contains a basic group, the addition salts thereof with a pharmaceutically acceptable acid.

**4.** Process according to claim 1 for the preparation of compounds of formula (I) in which B represents a CO-G group in which G represents straight-chain or branched lower alkyl substituted by a carboxy group, where applicable the isomers thereof, and also the addition salts thereof with a pharmaceutically acceptable base.

**5.** Process according to claim 1 for the preparation of compounds of formula (I) in which B represents a CO-G group in which G represents straight-chain or branched lower alkenyl substituted by a carboxy group, the isomers thereof, and also the addition salts thereof with a pharmaceutically acceptable base.

**6.** Process according to claim 1 for the preparation of compounds of formula (I) in which B represents a phenyl or naphthyl group substituted by a carboxylic group, and also the addition salts thereof with a pharmaceutically acceptable base.

**7.** Process according to either claim 1 or 2 for the preparation of compounds of formula (I) in which A and B together form a $CO(CH_2)_nCH(CH_3)$ group in which n is an integer 1, 2, 3 or 4, the CO group being bonded to the aromatic ring of the benzoxazolinone at the 5 or 6 position, and also the isomers thereof.

**8.** Process according to claim 1 for the preparation of compounds of formula (I) in which B represents a CO-G group in which G represents a heterocycle selected from furan and pyridine, and also, when G represents pyridine, the addition salts thereof with a pharmaceutically acceptable acid.

**9.** Process for the preparation of pharmaceutical compositions comprising a compound obtained in accordance with claim 1, an isomer thereof or an addition salt thereof with a pharmaceutically acceptable acid.

**10.** Process according to claim 9 for the preparation of pharmaceutical compositions useful in the treatment or prevention of cerebrovascular and peripheral arterial ischaemic accidents.

**Patentansprüche**
**Patentansprüche für folgende Vertragsstaaten : AT, BE, CH, DE, DK, FR, GB, IT, LI, LU, NL, SE**

**1.** Derivate der allgemeinen Formel (I):

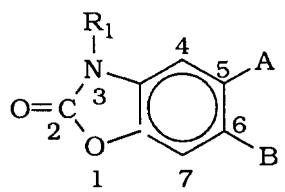

(I)

in der:

$R_1$ ein Wasserstoffatom oder eine Niedrigalkylgruppe,

A ein Wasserstoffatom und in diesem Fall B eine CO-G-Gruppe, worin G:

- entweder eine Heteroarylgruppe ausgewählt aus Furan, Indol, Pyrrol, Pyridin, Chinolin, Isochinolin, Benzofuran die gegebenenfalls durch eine oder mehrere Niedrigalkylgruppen oder Niedrigalkoxygruppen oder Halogenatome substituiert ist,
- oder eine geradkettige oder verzweigte, durch eine Carboxylgruppe substituierte Niedrigalkylgruppe,
- oder eine geradkettige oder verzweigte, durch eine Carboxylgruppe substituierte Niedrigalkenylgruppe,
- oder eine durch eine Carboxylgruppe substituierte Phenyl- oder Naphthylgruppe darstellt,
- oder A zusammen mit B eine Gruppe $CO(CH_2)_nCH(CH_3)$, worin n eine ganze Zahl mit einem Wert von 1, 2, 3 oder 4 darstellt und die CO-Gruppe in der 5- oder 6-Stellung des aromatischen Rings des Benzoxazolinons gebunden ist, bedeuten,

sowie gegebenenfalls deren Isomere und, wenn die Verbindung der Formel (I) eine Carbonsäuregruppe umfaßt, deren Additionssalze mit einer pharmazeutisch annehmbaren Base und, wenn die Verbindung der Formel (I) eine basische Gruppe aufweist, deren Additionssalze mit einer pharmazeutisch annehmbaren Säure, wobei es sich versteht, daß die Niedrigalkylgruppen, Niedrigalkoxygruppen und Niedrigalkenylgruppen für geradkettige oder verzweigte Gruppen mit 1 bis 6 Kohlenstoffatomen stehen.

**2.** Verbindungen nach Anspruch 1, worin B eine Gruppe CO-G darstellt, worin G eine aus Furan, Indol, Pyrrol, Pyridin, Chinolin, Isochinolin, Benzofuran, die gegebenenfalls durch eine oder mehrere Niedrigalkyl- oder Niedrigalkoxy-gruppen oder Halogenatome substituiert sind, ausgewählte Heteroarylgruppe darstellt, sowie, wenn G eine basische Gruppe aufweist, deren Additionssalze mit einer pharmazeutisch annehmbaren Säure.

**3.** Verbindungen nach Anspruch 1, worin B eine Gruppe CO-G bedeutet, worin G eine geradkettige oder verzweigte, durch eine Carboxylgruppe substituierte Niedrigalkylgruppe darstellt, sowie gegebenenfalls deren Isomere und deren Additionssalze mit einer pharmazeutisch annehmbaren Base.

**4.** Verbindungen nach Anspruch 1, worin B eine Gruppe CO-G bedeutet, worin G eine geradkettige oder verzweigte, durch eine Carboxylgruppe substituierte Niedrigalkenylgruppe darstellt, deren Isomeren

sowie deren Additionssalze mit einer pharmazeutisch annehmbaren Base.

**5.** Verbindungen nach Anspruch 1, worin B eine durch eine Carboxylgruppe substituierte Phenyl- oder Naphthyl-gruppe darstellt, sowie deren Additionssalze mit einer pharmazeutisch annehmbaren Base.

**6.** Verbindungen nach Anspruch 1, worin A zusammen mit B eine Gruppe $CO(CH_2)_nCH(CH_3)$ bildet, worin n eine ganze Zahl mit einem Wert von 1, 2, 3 oder 4 bedeutet und die Gruppe CO in der 5- oder 6-Stellung des aromatischen Rings des Benzoxazolinons gebunden Ist, sowie deren Isomere.

**7.** Verbindungen nach einem der Ansprüche 1 oder 2, worin B eine Gruppe CO-G bedeutet, worin G einen aus Furan oder Pyridin ausgewählten Heterocyclus darstellt, sowie, wenn G Pyridin bedeutet, deren Additionssalze mit einer pharmazeutisch annehmbaren Säure.

**8.** Verfahren zur Herstellung der Verbindungen der Formel (I) nach Anspruch 1, **dadurch gekennzeichnet**, daß man als Ausgangsmaterial ein Derivat der Formel (II) verwendet:

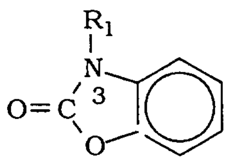

(II)

in der $R_1$ die bezüglich der Formel (I) angegebenen Bedeutungen besitzt, die man in Gegenwart von Dimethylformamid und Aluminiumchlorid mit:

a) entweder:

- einem Säurechlorid der Formel (III):

ClCOG (III)

in der:

G die bezüglich der Formel (I) angegebenen Bedeutungen besitzt, mit Ausnahme des Falles, da G eine durch eine Carboxylgruppe substituierte Niedrigalkenylgruppe darstellt,

- oder mit einem Säureanhydrid der Formel (IV):

$O(OCG)_2$ (IV)

in der G die bezüglich der Formel (I) angegebenen Bedeutungen besitzt,

behandelt zur Bildung eines Derivats der Formel (I/a):

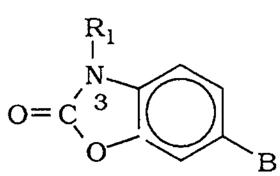

(I/a)

einem Sonderfall der Derivate der Formel (I), in der A ein Wasserstoffatom bedeutet und B die bezüglich der Formel (I) angegebenen Bedeutungen für den Fall besitzt, da A ein Wasserstoffatom darstellt,

welches Derivat der Formel (I/a) man erforderlichenfalls mit Hilfe einer klassischen Methode, ausgewählt aus Kristallisation oder Chromatographie, reinigt und das man gegebenenfalls in die Isomeren auftrennt und gewünschtenfalls, wenn B eine Carboxylgruppe aufweist, mit einer pharmazeutisch annehmbaren Base und, wenn B eine basische Gruppe aufweist, mit einer pharmazeutisch annehmbaren Säure in ein Salz überführt,

b) oder mit einem Säurechlorid der Formel (V):

$ClOC-(CH_2)_{n-1}CH = CH-CH_3$ (V)

in der n die bezüglich der Formel (I) angegebene Bedeutung besitzt, oder mit einem Derivat der Formel:

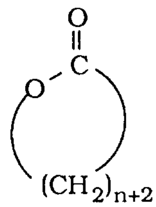

in der n die bezüglich der Formel (I) angegebenen Bedeutungen besitzt, behandelt, so daß man eine Mischung der Derivate der Formel (I/b) erhält:

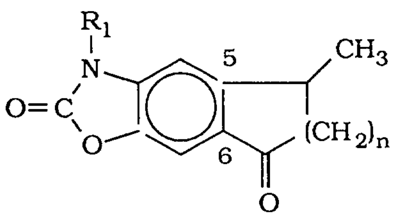

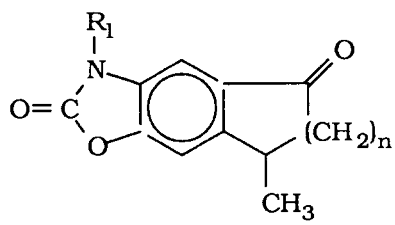

(I/b)

in der $R_1$ und n die bezüglich der Formel (I) angegebenen Bedeutungen besitzen, einem Sonderfall der Derivate der Formel (I), in der A zusammen mit B eine Gruppe $CO(CH_2)_n)CHCH_3$ bildet, worin n die bezüglich der Formel (I) angegebene Bedeutung besitzt, die Gruppe CO entweder in der 5-Stellung oder in der 6-Stellung des Benzoxazolinons gebunden ist, welche Derivate der Formel (I/b) man trennt und mit Hilfe einer oder mehrerer Methoden ausgewählt aus Kristallisation oder Chromatographie reinigt.

**9.** Verfahren zur Synthese der Verbindungen der Formel (I/c) nach Anspruch 1:

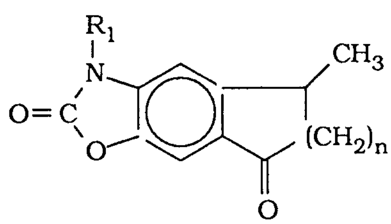

(I/c)

in der $R_1$ und n die bezüglich der Formel (I) angegebenen Bedeutungen besitzen, **dadurch gekennzeichnet**, daß man ein Derivat der Formel (IV):

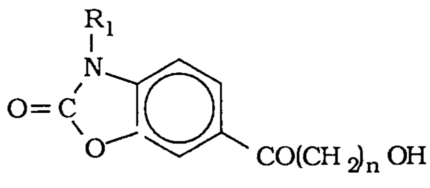

(IV)

in der $R_1$ und n die bezüglich der Formel (I) angegebenen Bedeutungen besitzen, der Einwirkung von Aluminiumchlorid in Gegenwart von Dimethylformamid unterwirft zur Bildung eines Derivats der Formel (I/c), welches man gewünschtenfalls mit Hilfe einer oder mehrerer Methoden, ausgewählt aus Kristallisation und/oder Chromatographie, reinigt.

**10.** Pharmazeutische Zubereitung enthaltend als Wirkstoff mindestens eine Verbindung nach einem der Ansprüche 1 bis 7 in Kombination mit einem oder mehreren inerten, nichttoxischen, pharmazeutisch annehmbaren Trägermaterialien oder Bindemitteln.

**11.** Pharmazeutische Zubereitung nach Anspruch 10 enthaltend mindestens einen Wirkstoff nach einem der Ansprüche 1 bis 7 zur Behandlung und zur Vorbeugung von peripheren und zerebrovaskulären arteriellen ischämischen Unfällen.

**Patentansprüche für folgenden Vertragsstaat : ES**

**1.** Verfahren zur Herstellung von Derivaten der Formel (I):

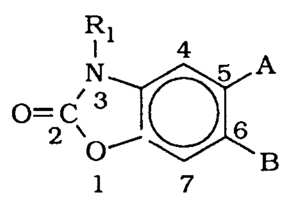

(I)

in der:

R$_1$ ein Wasserstoffatom oder eine Niedrigalkylgruppe,

A ein Wasserstoffatom und in diesem Fall B eine CO-G-Gruppe, worin G:

- entweder eine Heteroarylgruppe ausgewählt aus Furan, Indol, Pyrrol, Pyridin, Chinolin, Isochinolin, Benzofuran, die gegebenenfalls durch eine oder mehrere Niedrigalkylgruppen oder Niedrigalkoxygruppen oder Halogenatome substituiert ist,
- oder eine geradkettige oder verzweigte, durch eine Carboxylgruppe substituierte Niedrigalkylgruppe,
- oder eine geradkettige oder verzweigte, durch eine Carboxylgruppe substituierte Niedrigalkenylgruppe,
- oder eine durch eine Carboxylgruppe substituierte Phenyl- oder Naphthylgruppe darstellt,
- oder A zusammen mit B eine Gruppe $CO(CH_2)_nCH(CH_3)$, worin n eine ganze Zahl mit einem Wert von 1, 2, 3 oder 4 darstellt und die CO-Gruppe in der 5- oder 6-Stellung des aromatischen Rings des Benzoxazolinons gebunden ist, bedeuten,

  sowie gegebenenfalls von deren Isomeren und, wenn die Verbindung der Formel (I) eine Carbonsäuregruppe umfaßt, von deren Additionssalzen mit einer pharmazeutisch annehmbaren Base und, wenn die Verbindung der Formel (I) eine basische Gruppe aufweist, von deren Additionssalzen mit einer pharmazeutisch annehmbaren Säure, wobei es sich versteht, daß die Niedrigalkylgruppen, Niedrigalkoxygruppen und Niedrigalkenylgruppen für geradkettige oder verzweigte Gruppen mit 1 bis 6 Kohlenstoffatomen stehen,

**dadurch gekennzeichnet**, daß man als Ausgangsmaterial ein Derivat der Formel (II) verwendet:

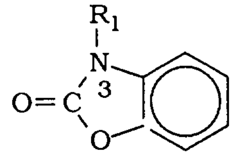

(II)

in der R$_1$ die bezüglich der Formel (I) angegebenen Bedeutungen besitzt, die man in Gegenwart von Dimethylformamid und Aluminiumchlorid mit:

a) entweder:

- einem Säurechlorid der Formel (III):

ClCOG (III)

in der:
G die bezüglich der Formel (I) angegebenen Bedeutungen besitzt, mit Ausnahme des Falles, da G eine durch eine Carboxylgruppe substituierte Niedrigalkenylgruppe darstellt,

- oder mit einem Säureanhydrid der Formel (IV):

$O(OCG)_2$ (IV)

in der G die bezüglich der Formel (I) angegebenen Bedeutungen besitzt,
behandelt zur Bildung eines Derivats der Formel (I/a):

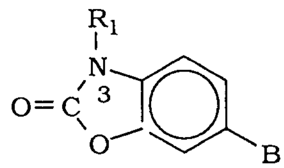

(I/a)

einem Sonderfall der Derivate der Formel (I), in der A ein Wasserstoffatom bedeutet und B die bezüglich der Formel (I) angegebenen Bedeutungen für den Fall besitzt, da A ein Wasserstoffatom darstellt,
welches Derivat der Formel (I/a) man erforderlichenfalls mit Hilfe einer klassischen Methode, ausgewählt aus Kristallisation oder Chromatographie, reinigt und das man gegebenenfalls in die Isomeren auftrennt und gewünschtenfalls, wenn B eine Carboxylgruppe aufweist, mit einer pharmazeutisch annehmbaren Base und, wenn B eine basische Gruppe aufweist, mit einer pharmazeutisch annehmbaren Säure in ein Salz überführt,
b) oder mit einem Säurechlorid der Formel (V):

$ClOC - (CH_2)_{n-1}CH = CH - CH_3$ (V)

in der n die bezüglich der Formel (I) angegebene Bedeutung besitzt,
oder mit einem Derivat der Formel:

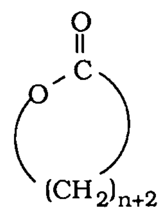

in der n die bezüglich der Formel (I) angegebenen Bedeutungen besitzt, behandelt,
so daß man eine Mischung der Derivate der Formel (I/b) erhält:

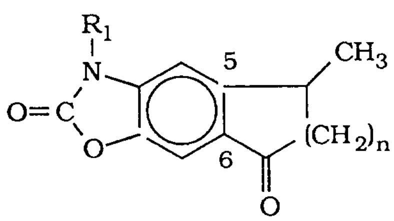

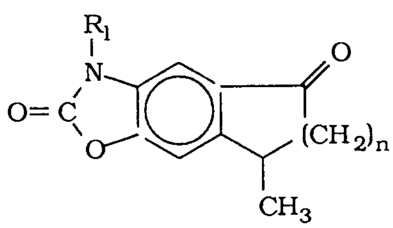

(I/b)

in der $R_1$ und n die bezüglich der Formel (I) angegebenen Bedeutungen besitzen, einem Sonderfall der Derivate der Formel (I), in der A zusammen mit B eine Gruppe $CO(CH_2)_n)CHCH_3$ bildet, worin n die bezüglich der Formel (I) angegebene Bedeutung besitzt, die Gruppe CO entweder in der 5-Stellung oder in der 6-Stellung des Benzoxazolinons gebunden ist, welche Derivate der Formel (I/b)

man trennt und mit Hilfe einer oder mehrerer Methoden, ausgewählt aus Kristallisation oder Chromatographie, reinigt.

2. Verfahren nach Anspruch 1 zur Synthese von Verbindungen der Formel (I/c):

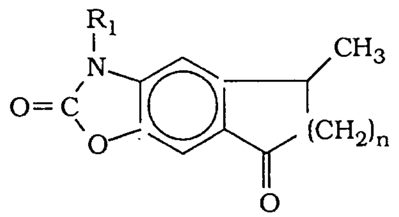

(I/c)

in der $R_1$ und n die bezüglich der Formel (I) in Anspruch 1 angegebenen Bedeutungen besitzen, **dadurch gekennzeichnet**, daß man ein Derivat der Formel (IV):

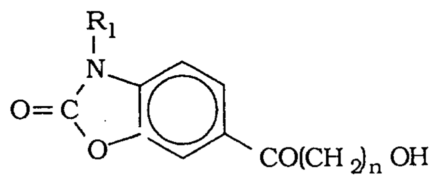

(IV)

in der $R_1$ und n die bezüglich der Formel (I) angegebenen Bedeutungen besitzen, der Einwirkung von Aluminiumchlorid in Gegenwart von Dimethylformamid unterwirft zur Bildung eines Derivats der Formel (I/c), welches man gewünschtenfalls mit Hilfe einer oder mehrerer Methoden, ausgewählt aus Kristallisation und/oder Chromatographie, reinigt.

3. Verfahren nach Anspruch 1 zur Herstellung von Verbindungen der Formel (I), worin B eine Gruppe CO-G bedeutet, worin G eine aus Furan, Indol, Pyrrol, Pyridin, Chinolin, Isochinolin und Benzofuran ausgewählte Heteroarylgruppe, die gegebenenfalls durch eine oder mehrere Niedrigalkyl- oder Niedrigalkoxy-gruppen oder Halogenatome substituiert sind, darstellt sowie, wenn G eine basische Gruppe umfaßt, von deren Additionssalzen mit einer pharmazeutisch annehmbaren Säure.

4. Verfahren nach Anspruch 1 zur Herstellung von Verbindungen der Formel (I), worin B eine Gruppe CO-G bedeutet, worin G eine durch eine Carboxylgruppe substituierte geradkettige oder verzweigte Niedrigalkylgruppe darstellt, sowie gegebenenfalls von deren Isomeren sowie von deren Additionssalzen mit einer pharmazeutisch annehmbaren Base.

5. Verfahren nach Anspruch 1 zur Herstellung von Verbindungen der Formel (I), worin B eine Gruppe CO-G bedeutet, worin G eine durch eine Carboxylgruppe substituierte geradkettige oder verzweigte Niedrigalkenylgruppe darstellt, von deren Isomeren sowie von deren Additionssalzen mit einer pharmazeutisch annehmbaren Base.

6. Verfahren nach Anspruch 1 zur Herstellung von Verbindungen der Formel (I), worin B eine durch eine Carboxylgruppe substituierte Phenyl- oder Naphthylgruppe bedeutet, sowie von deren Additionssalzen mit einer pharmazeutisch annehmbaren Base.

7. Verfahren nach einem der Ansprüche 1 oder 2 zur Herstellung von Verbindungen der Formel (I), worin A zusammen mit B eine Gruppe $CO(CH_2)_nCH(CH_3)$ bildet, worin n eine ganze Zahl mit einem Wert von 1, 2, 3 oder 4 darstellt und die Gruppe CO in der 5- oder 6-Stellung des aromatischen Rings des Benzoxazolinons gebunden ist, sowie von deren Isomeren.

8. Verfahren nach Anspruch 1 zur Herstellung von Verbindungen der Formel (I), worin B eine Gruppe CO-G bedeutet, worin G einen aus Pyridin oder Furan ausgewählten Heterocyclus darstellt, sowie, wenn G

Pyridin bedeutet, von deren Additionssalzen mit einer pharmazeutisch annehmbaren Säure.

**9.** Verfahren zur Herstellung von pharmazeutischen Zubereitungen enthaltend eine nach Anspruch 1 erhaltene Verbindung, deren Isomere oder deren Additionssalze mit einer pharmazeutisch annehmbaren Säure.

**10.** Verfahren nach Anspruch 9 zur Herstellung von pharmazeutischen Zubereitungen zur Behandlung oder zur Vorbeugung von peripheren und zerebrovaskulären arteriellen ischämischen Unfällen.

**Patentansprüche für folgenden Vertragsstaat : GR**

**1.** Verfahren zur Herstellung von Derivaten der Formel (I):

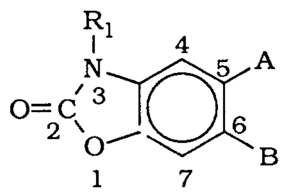

(I)

in der:

$R_1$ ein Wasserstoffatom oder eine Niedrigalkylgruppe,

A ein Wasserstoffatom und in diesem Fall B eine CO-G-Gruppe, worin G:

- entweder eine Heteroarylgruppe ausgewählt aus Furan, Indol, Pyrrol, Pyridin, Chinolin, Isochinolin, Benzofuran, die gegebenenfalls durch eine oder mehrere Niedrigalkylgruppen oder Niedrigalkoxygruppen oder Halogenatome substituiert ist,
- oder eine geradkettige oder verzweigte, durch eine Carboxylgruppe substituierte Niedrigalkylgruppe,
- oder eine geradkettige oder verzweigte, durch eine Carboxylgruppe substituierte Niedrigalkenylgruppe,
- oder eine durch eine Carboxylgruppe substituierte Phenyl- oder Naphthylgruppe darstellt,
- oder A zusammen mit B eine Gruppe $CO(CH_2)_nCH(CH_3)$, worin n eine ganze Zahl mit einem Wert von 1, 2, 3 oder 4 darstellt und die CO-Gruppe in der 5- oder 6-Stellung des aromatischen Rings des Benzoxazolinons gebunden ist, bedeuten,

sowie gegebenenfalls von deren Isomeren und, wenn die Verbindung der Formel (I) eine Carbonsäuregruppe umfaßt, von deren Additionssalzen mit einer pharmazeutisch annehmbaren Base und, wenn die Verbindung der Formel (I) eine basische Gruppe aufweist, von deren Additionssalzen mit einer pharmazeutisch annehmbaren Säure, wobei es sich versteht, daß die Niedrigalkylgruppen, Niedrigalkoxygruppen und Niedrigalkenylgruppen für geradkettige oder verzweigte Gruppen mit 1 bis 6 Kohlenstoffatomen stehen,

**dadurch gekennzeichnet**, daß man als Ausgangsmaterial ein Derivat der Formel (II) verwendet:

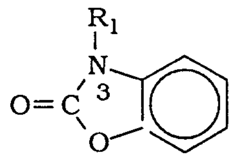

(II)

in der $R_1$ die bezüglich der Formel (I) angegebenen Bedeutungen besitzt, die man in Gegenwart von Dimethylformamid und Aluminiumchlorid mit:

a) entweder:

- einem Säurechlorid der Formel (III):

ClCOG (III)

in der:

G die bezüglich der Formel (I) angegebenen Bedeutungen besitzt, mit Ausnahme des Falles, da G eine durch eine Carboxylgruppe substituierte Niedrigalkenylgruppe darstellt,

- oder mit einem Säureanhydrid der Formel (IV):

$O(OCG)_2$ (IV)

in der G die bezüglich der Formel (I) angegebenen Bedeutungen besitzt,

behandelt zur Bildung eines Derivats der Formel (I/a):

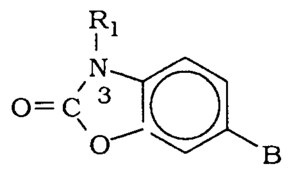

(I/a)

einem Sonderfall der Derivate der Formel (I), in der A ein Wasserstoffatom bedeutet und B die bezüglich der Formel (I) angegebenen Bedeutungen für den Fall besitzt, da A ein Wasserstoffatom darstellt,

welches Derivat der Formel (I/a) man erforderlichenfalls mit Hilfe einer klassischen Methode, ausgewählt aus Kristallisation oder Chromatographie, reinigt und das man gegebenenfalls in die Isomeren auftrennt und gewünschtenfalls, wenn B eine Carboxylgruppe aufweist, mit einer pharmazeutisch annehmbaren Base und, wenn B eine basische Gruppe aufweist, mit einer pharmazeutisch annehmbaren Säure in ein Salz überführt,

b) oder mit einem Säurechlorid der Formel (V):

$ClOC - (CH_2)_{n-1}CH = CH - CH_3$ (V)

in der n die bezüglich der Formel (I) angegebene Bedeutung besitzt,

oder mit einem Derivat der Formel:

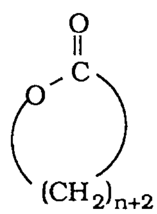

in der n die bezüglich der Formel (I) angegebenen Bedeutungen besitzt, behandelt,

so daß man eine Mischung der Derivate der Formel (I/b) erhält:

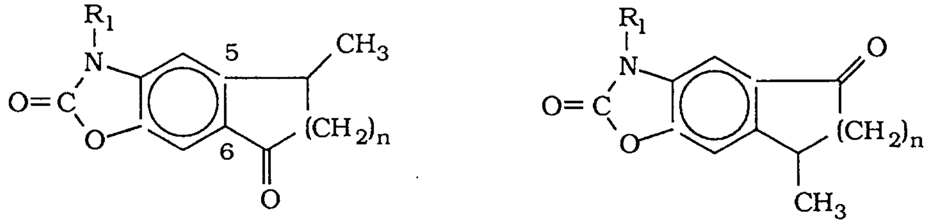

(I/b)

in der $R_1$ und n die bezüglich der Formel (I) angegebenen Bedeutungen besitzen, einem Sonderfall der Derivate der Formel (I), in der A zusammen mit B eine Gruppe $CO(CH_2)_n)CHCH_3$ bildet, worin n die bezüglich der Formel (I) angegebene Bedeutung besitzt, die Gruppe CO entweder in der 5-Stellung oder in der 6-Stellung des Benzoxazolinons gebunden ist, welche Derivate der Formel (I/b)

man trennt und mit Hilfe einer oder mehrerer Methoden, ausgewählt aus Kristallisation oder Chromatographie, reinigt.

2. Verfahren nach Anspruch 1 zur Synthese von Verbindungen der Formel (I/c):

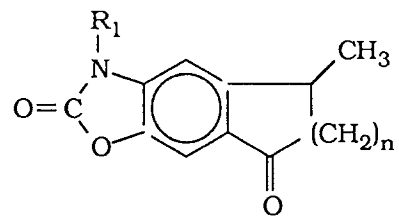

(I/c)

in der $R_1$ und n die bezüglich der Formel (I) in Anspruch 1 angegebenen Bedeutungen besitzen, **dadurch gekennzeichnet**, daß man ein Derivat der Formel (IV):

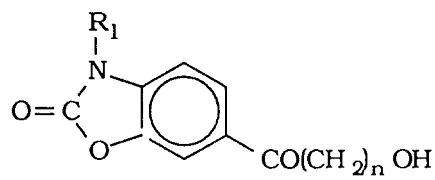

(IV)

in der $R_1$ und n die bezüglich der Formel (I) angegebenen Bedeutungen besitzen, der Einwirkung von Aluminiumchlorid in Gegenwart von Dimethylformamid unterwirft zur Bildung eines Derivats der Formel (I/c), welches man gewünschtenfalls mit Hilfe einer oder mehrerer Methoden, ausgewählt aus Kristallisation und/oder Chromatographie, reinigt.

3. Verfahren nach Anspruch 1 zur Herstellung von Verbindungen der Formel (I), worin B eine Gruppe CO-G bedeutet, worin G eine aus Furan, Indol, Pyrrol, Pyridin, Chinolin, Isochinolin und Benzofuran ausgewählte Heteroarylgruppe, die gegebenenfalls durch eine oder mehrere Niedrigalkyl- oder Niedrigalkoxy-gruppen oder Halogenatome substituiert sind, darstellt sowie, wenn G eine basische Gruppe umfaßt, von deren Additionssalzen mit einer pharmazeutisch annehmbaren Säure.

4. Verfahren nach Anspruch 1 zur Herstellung von Verbindungen der Formel (I), worin B eine Gruppe CO-G bedeutet, worin G eine durch eine Carboxylgruppe substituierte geradkettige oder verzweigte Niedrigalkylgruppe darstellt, sowie gegebenenfalls von deren Isomeren sowie von deren Additionssalzen mit einer pharmazeutisch annehmbaren Base.

5. Verfahren nach Anspruch 1 zur Herstellung von Verbindungen der Formel (I), worin B eine Gruppe CO-G bedeutet, worin G eine durch eine Carboxylgruppe substituierte geradkettige oder verzweigte Niedrigalkenylgruppe darstellt, von deren Isomeren sowie von deren Additionssalzen mit einer pharmazeutisch annehmbaren Base.

6. Verfahren nach Anspruch 1 zur Herstellung von Verbindungen der Formel (I), worin B eine durch eine Carboxylgruppe substituierte Phenyl- oder Naphthylgruppe bedeutet, sowie von deren Additionssalzen mit einer pharmazeutisch annehmbaren Base.

7. Verfahren nach einem der Ansprüche 1 oder 2 zur Herstellung von Verbindungen der Formel (I), worin A zusammen mit B eine Gruppe $CO(CH_2)_nCH(CH_3)$ bildet, worin n eine ganze Zahl mit einem Wert von 1, 2, 3 oder 4 darstellt und die Gruppe CO in der 5- oder 6-Stellung des aromatischen Rings des Benzoxazolinons gebunden ist, sowie von deren Isomeren.

8. Verfahren nach Anspruch 1 zur Herstellung von Verbindungen der Formel (I), worin B eine Gruppe CO-G bedeutet, worin G einen aus Pyridin oder Furan ausgewählten Heterocyclus darstellt, sowie, wenn G Pyridin bedeutet, von deren Additionssalzen mit einer pharmazeutisch annehmbaren Säure.

9. Verfahren zur Herstellung von pharmazeutischen Zubereitungen enthaltend eine nach Anspruch 1 erhaltene Verbindung, deren Isomere oder deren Additionssalze mit einer pharmazeutisch annehmbaren Säure.

10. Verfahren nach Anspruch 9 zur Herstellung von pharmazeutischen Zubereitungen zur Behandlung oder zur Vorbeugung von peripheren und zerebrovaskulären arteriellen ischämischen Unfällen.